(19) Europäisches Patentamt
European Patent Office
Office européen des brevets

(11) **EP 2 859 599 B1**

(12) **EUROPEAN PATENT SPECIFICATION**

(45) Date of publication and mention
of the grant of the patent:
**11.11.2020 Bulletin 2020/46**

(51) Int Cl.:
***H01L 41/257*** *(2013.01)*

(21) Application number: **13803506.8**

(22) Date of filing: **11.06.2013**

(86) International application number:
**PCT/US2013/045147**

(87) International publication number:
**WO 2013/188380 (19.12.2013 Gazette 2013/51)**

(54) **PIEZOELECTRIC COMPOSITES AND METHODS OF MAKING**

PIEZOELEKTRISCHE VERBUNDSTOFFE UND VERFAHREN ZUR HERSTELLUNG

COMPOSITES PIÉZOÉLECTRIQUES ET PROCÉDÉS DE FABRICATION DESDITS COMPOSITES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.06.2012 US 201261658727 P
10.04.2013 US 201361810458 P**

(43) Date of publication of application:
**15.04.2015 Bulletin 2015/16**

(73) Proprietor: **The University of Kansas
Lawrence, KS 66045 (US)**

(72) Inventors:
 • **FRIIS, Elizabeth, Annamaria**
 **Lawrence, KS 66047 (US)**
 • **DOMANN, John, Patrick**
 **Los Angeles, CA 90049 (US)**

(74) Representative: **Nottrott, Stephanie et al
Patentanwälte
Isenbruck Bösl Hörschler PartG mbB
Prinzregentenstraße 68
81675 München (DE)**

(56) References cited:
 WO-A1-2010/147470  US-A- 4 407 054
 US-A1- 2004 000 661  US-A1- 2004 232 803
 US-A1- 2010 104 876  US-A1- 2011 118 852
 US-B2- 7 615 373

• VAN DEN ENDE D ET AL: "Improving the d33 and
g33 properties of 0-3 piezoelectric composites by
dielectrophoresis", JOURNAL OF APPLIED
PHYSICS, AMERICAN INSTITUTE OF PHYSICS,
US, vol. 107, no. 2, 25 January 2010 (2010-01-25),
pages 24107-24107, XP012133218, ISSN:
0021-8979, DOI: 10.1063/1.3291131
• YUWANG HAN ET AL: "Thermal conductivity
enhancement of BN/silicone composites cured
under electric field: Stacking of shape, thermal
conductivity, and particle packing structure
anisotropies", THERMOCHIMICA ACTA,
ELSEVIER SCIENCE PUBLISHERS,
AMSTERDAM, NL, vol. 529, 22 November 2011
(2011-11-22), pages 68-73, XP028395676, ISSN:
0040-6031, DOI: 10.1016/J.TCA.2011.11.029
[retrieved on 2011-12-01]
• VAN DEN ENDE ET AL.: 'Improving the d33 and
g33 Properties of 0-3 Piezoelectric Composites
by Dielectrophoresis.' JOURNAL OF APPLIED
PHYSICS vol. 107, 25 January 2010, XP012133218
Retrieved from the Internet:
<URL:http://www.google.com/url?
sa=t&rct=j8q=&esrc=s&source=web&cd=18ved
=OC DQOFjAA&url=http%3A%2F%
2Frepository.tudelft.nl%2Fassets%2Fuuid%3A0
t938f54-7f7e-4d30-8c9c-6654d23fdf87%
2FvandenEnde_2010.pdf8ei=69ZpUsnCKai52w
Wb2Y DOAg&usg=AFQjCNEOcFaAOuqwhahN
UdBXdqYEivY6ag8sig2=J5bd1SV3UMU323BOs
OmORA& bvm=bv.551> [retrieved on 2013-10-22]

EP 2 859 599 B1

- BOWEN ET AL.: 'Tunable Electric Field Processing of Composite Materials.' JOURNAL OF INTELLIGENT MATERIAL SYSTEMS AND STRUCTURES. vol. 6, no. 2, March 1995, XP008175851 Retrieved from the Internet: <URL:http://jim.sagepub.com/content/6/2/159 .short> [retrieved on 2013-10-24]
- DATABASE INSPEC [Online] THE INSTITUTION OF ELECTRICAL ENGINEERS, STEVENAGE, GB; April 1996 (1996-04), LOUIS P ET AL: "Computer simulation of spatial arrangement and connectivity of particles in three-dimensional microstructure: application to model electrical conductivity of polymer matrix composite", Database accession no. 5240618 & ACTA MATERIALIA ELSEVIER USA, vol. 44, no. 4, 25 January 2010 (2010-01-25), pages 1519-1528, ISSN: 1359-6454

**Description**

BACKGROUND OF THE INVENTION

Field of the Invention

The present application relates to piezoelectric composites comprising polymerizable matrices and piezoelectric particles dispersed therein. Suitably the composites are useful as tissue-stimulating materials, including spinal implants. The present application also relates to methods of making piezoelectric composites.

Background of the Invention

Electrical stimulation has proven to be an effective therapy to increase the success rate of spinal fusions, especially in the difficult-to-fuse population. However, in its current form, it is hampered by limitations such as the need for a battery pack or a separate implantable device to provide power, and reliance on user compliance for externally worn devices. An alternative treatment to aid in bone growth stimulation involves the use of growth factors such as bone morphogenic protein (BMP). However, studies on BMP have shown that it has a substantial risk for complication, including ectopic bone formation. The growth of bone spurs near the spinal canal is also of concern for anyone receiving this treatment. Some studies also suggest a carcinogenic effect related to the use of BMP.

One potential method by which electrical stimulation can be generated is through the use of piezoelectric materials. Piezoelectric materials are a class of ferroelectrics characterized by a net polarization, often due to a non-centro-symmetric crystalline structure. As a result, piezoelectric materials respond to stress with the generation of a net surface charge. Conversely, piezoelectric materials can be strained with the application of an electric field. Similar to high performance dielectric materials, piezoceramics are the most often used piezoelectric material, though they tend to be stiff and brittle.

In spinal fusions, the use of such materials has generally been hampered by limitations on the size and shape of current piezoelectric structures as a result of the constraints of the manufacturing process, specifically, the need to pole a composite to induce a net piezoelectric behavior. This procedure requires excessively high electric field strengths that can therefore bring about material failure and limits the choice of available materials to those with a high dielectric strength. This, in turn, limits the total size that the composition can achieve. In the case of spinal implants, thicknesses on the order of 10-20 mm or greater are generally required, which is difficult to obtain with current methods. Examples of prior art may be found in "Improving the d33 and g33 properties of 0-3 piezoelectric composites by dielectrophoresis", Journal of Applied Physics, Vol. 107, Nr. 2, Pg. 24107-1 - 24107-8, and in patent document US4407054.

SUMMARY OF PREFERRED EMBODIMENTS

Thus, there is a need for methods that can produce piezoelectric composites having suitable physical characteristics and also optimized electrical stimulatory properties.

The present application provides piezoelectric composites, including tissue-stimulating composites, as well as methods of making such composites, that meet these needs.

An embodiment of the invention, which is a method of making a piezoelectric composite is given in claim 1. The methods suitably comprise preparing a thermoset, thermoplastic or thermoset/thermoplastic, or copolymer polymerizable matrix, dispersing a plurality of piezoelectric particles in the polymerizable matrix to generate a dispersion, shaping the dispersion, inducing an electric polarization in the piezoelectric particles in the shaped dispersion, wherein at least 40% of the piezoelectric particles form chains as a result of the induction of the electric polarization, and curing the dispersion to generate the spinal implant.

In embodiments, the shaping comprises injection molding, extrusion, compression molding, blow molding or thermoforming.

Suitably, the piezoelectric particles exhibit a Perovskite crystalline structure. Exemplary piezoelectric particles include, but are not limited to, particles of barium titanate, particles of hydroxyapatite, particles of apatite, particles of lithium sulfate monohydrate, particles of sodium potassium niobate, particles of quartz, particles of lead zirconium titanate (PZT), particles of tartaric acid and poly(vinylidene difluoride) fibers.

In embodoiments, the inducing an electric polarization comprises applying an electric field in a direction to the shaped dispersion.

Suitably, the inducing an electric polarization comprises applying a hydrostatic pressure to the shaped dispersion or changing the temperature of the shaped dispersion. In embodiments, prior to the curing, the methods further comprise applying an electric field in a direction to the shaped dispersion.

Suitably applying an electric field comprises applying a field with a frequency of about 1 kHz to about 10 kHz and a field strength of about 1 Volt/mm to about 1 kV/mm. In additional embodiments, the applying an electric field comprises applying a field with a frequency of about 1 Hz to about 100 Hz and a field strength of about 1 Volt/mm to about 1 kV/mm.

Suitably, the inducing in occurs before the applying an electric field, or the inducing in can occur after the applying an electric field, or the inducing and the applying an electric field occur simultaneously.

In embodiments, the electric field is applied at the same frequency with a cyclic hydrostatic pressure.

In suitable embodiments, the curing comprises cooling, UV curing, heat accelerated curing or compression curing the dispersion.

In exemplary embodiments, the chains have a random orientation. In other embodiments, at least about 10% of the chains are aligned to within about ±10 degrees of the direction of the applied electric field, more suitably at least about 50% of the chains are aligned to within about ±10 degrees of the

Also provided are spinal implants prepared by the methods described herein.

In embodiments, spinal implants are provided comprising a polymer matrix and a plurality of piezoelectric particles, wherein at least 40% of the piezoelectric particles are in the form of chains, and the implant is a 1-3 composite.

In embodiments, at least about 10% of the chains are aligned to within ±10 degrees of each other, or at least about 50% of the chains are aligned to within ±10 degrees of each other.

Suitably, the piezoelectric particles exhibit a Perovskite crystalline structure. In embodiments, the piezoelectric particles are selected from the group consisting of particles of barium titanate, particles of hydroxyapatite, particles of apatite, particles of lithium sulfate monohydrate, particles of sodium potassium niobate, particles of quartz, particles of lead zirconium titanate (PZT), particles of tartaric acid and poly(vinylidene difluoride) fibers.

In suitable embodiments, the implant generates a current density of between about 1 to about 250 micro-amps/cm$^2$ when compressed.

In embodiments, methods of making a piezoelectric composite are provided. Suitably, the methods comprise preparing a polymerizable matrix, dispersing a plurality of piezoelectric particles in the polymerizable matrix to generate a dispersion, shaping the dispersion, inducing an electric polarization in the piezoelectric particles in the shaped dispersion, wherein at least 40% of the piezoelectric particles form chains as a result of the induction of the electric polarization, and curing the dispersion.

Suitably, the polymerizable matrix comprises a thermoset polymer, copolymer and/or monomer, a thermoplastic polymer, copolymer and/or monomer or a thermoset/thermoplastic polymer or copolymer blend.

In exemplary embodiments, piezoelectric particles for use in the methods and compositions described herein exhibit a Perovskite crystalline structure. Suitable piezoelectric particles include, but are not limited to, particles of barium titanate, particles of hydroxyapatite, particles of apatite, particles of lithium sulfate monohydrate, particles of sodium potassium niobate, particles of quartz, particles of lead zirconium titanate (PZT), particles of tartaric acid and poly(vinylidene difluoride) fibers.

Suitably, shaping the dispersion comprises injection molding, extrusion, compression molding, blow molding or thermoforming.

In embodiments, inducing an electric polarization comprises applying an electric field in a direction to the shaped dispersion. In other embodiments, inducing an electric polarization comprises applying a hydrostatic pressure to the shaped dispersion or changing the temperature of the shaped dispersion. In additional embodiments, an electric field can be applied to the shaped dispersion in combination with the application of the hydrostatic pressure or change in temperature. This field can be applied before, after or simultaneously with the induction of the electric polarization. In embodiments, the electric field is applied at the same frequency, and can be in phase, with a cyclic hydrostatic pressure.

In embodiments, the electric field has a frequency of about 1 kHz to about 10 kHz and a field strength of about 1 Volt/mm to about 1kVolt/mm. In other embodiments, the electric field comprises a field with a frequency of about 1 Hz to about 100 Hz and a field strength of about 1 Volt/mm to about 1 kVolt/mm.

In embodiments, the curing process comprises cooling, UV curing, heat accelerated curing or compression curing the dispersion.

Suitably, the chains that are formed in the composite have a random orientation. In other embodiments, at least about 10% of the chains are aligned to within about ±10 degrees of the direction of the applied electric field, more suitably at least about 50% of the chains are aligned to within about ±10 degrees of the direction of the applied electric field.

Also provided are methods of making tissue-stimulating piezoelectric composites. The methods suitably comprise preparing a thermoset, thermoplastic, thermoset/thermoplastic (or copolymer) polymerizable matrix, dispersing a plurality of piezoelectric particles in the polymerizable matrix to generate a dispersion, shaping the dispersion, inducing an electric polarization in the piezoelectric particles in the shaped dispersion, wherein at least 40% of the piezoelectric particles form chains as a result of the induction of the electric polarization, and curing the dispersion.

Also provided are piezoelectric composites and tissue-stimulating piezoelectric composites prepared by the methods described throughout.

In embodiments, piezoelectric composites comprising a polymer matrix and a plurality of piezoelectric particles are provided. Suitably at least 40% of the piezoelectric particles are in the form of chains and the composite has at least one dimension of 5 mm or greater.

In embodiments, the composites are 1-3 composites. Suitably, the composites provided herein generate a current density of between about 1 to about 250 microamps/cm$^2$ when compressed.

In further embodiments, the composites are 1-3 composites. Suitably, the composites provided herein possess a piezoelectric charge coefficient $d_{33}$ of the composite between 1% and 100% of the bulk piezoelectric charge coefficient from which the composite is created.

Suitably, the composites provided herein possess a dielectric constant $\varepsilon_{33}$ of the composite between 1% and 100% of the bulk dielectric constant of the polymerizable matrix from which the composite is created.

Suitably, the composites provided herein possess a piezoelectric voltage constant $g_{33}$ of the composite between 1% and 1,000% of the bulk piezoelectric voltage coefficient from which the composite is created.

Also provided are tissue-stimulating piezoelectric composites comprising a polymer matrix and a plurality of piezoelectric particles. Suitably, at least 40% of the piezoelectric particles are in the form of chains, and the composite is a 1-3 composite.

Further embodiments, features, and advantages of the embodiments, as well as the structure and operation of the various embodiments, are described in detail below with reference to accompanying drawings.

BRIEF DESCRIPTION OF THE FIGURES

FIGs. 1A and 1B show representations of 0-3 and 1-3 structured composites, respectively.
FIGs. 2A and 2B show an exemplary method of making the piezoelectric composites described herein.
FIGs. 3A and 3B show representations of dielectric force (3A) on particles and piezoelectric force (3B) on particles, respectively.
FIG. 4A shows an exemplary manufacturing set-up for use with dielectrophoretic (DEP) formation of piezoelectric composites described herein.
FIG. 4B shows an exemplary manufacturing set-up for use with piezoelectrophoretic (PEP) formation of piezoelectric composites described herein.

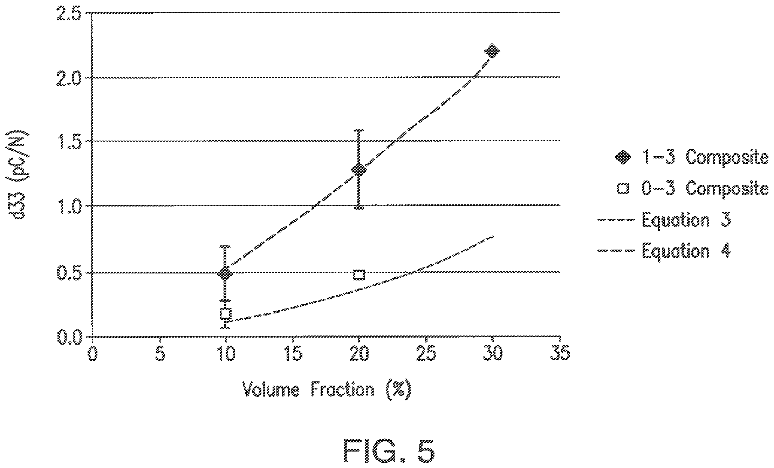

FIG. 5 shows the piezoelectric charge coefficient for 0-3 and 1-3 structure composites in accordance with embodiments described herein.
FIG. 6 shows a piezoelectric composite circuit model.
FIG. 7 shows a lumped parameters model of a mechanical system of the circuit model.
FIG. 8 shows model results for peak power versus thickness.
FIG. 9 shows model results for peak power versus cross-sectional area.
FIG. 10 shows model results for peak power versus fiber aspect ratio.
FIG. 11 shows model results for peak power versus volume fraction of the fibers and load resistance for a set implant geometry.
FIG. 12 shows model results for peak power versus load resistance for PZT and BaTiO$_3$.

DETAILED DESCRIPTION OF PREFERRED EMBODIMENTS

It should be appreciated that the particular implementations shown and described herein are examples and are not intended to otherwise limit the scope of the application in any way.

As used in this specification, the singular forms "a," "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

Technical and scientific terms used herein have the meaning commonly understood by one of skill in the art to which the present application pertains, unless otherwise defined. Reference is made herein to various methodologies and materials known to those of ordinary skill in the art.

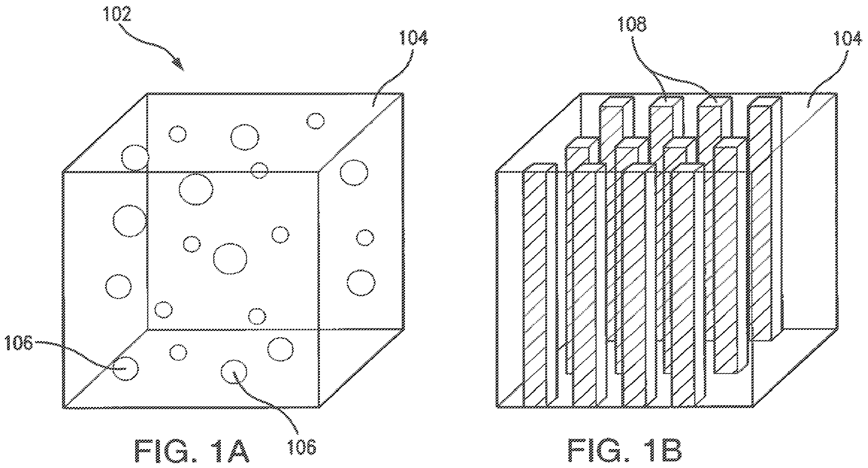

Composite matrices with 0-3 connectivity 102 are typically comprised of particles 106 randomly dispersed within a matrix 104 (FIG. 1A). The matrix 104 is connected to itself in all three spatial directions, while the particles 106 lack contact. As such, effective medium (EM) theory portrays the bulk, or apparent properties of these composites as isotropic. Manufacturing a 0-3 composite is a straightforward process that entails mixing small particle inclusions into a matrix until evenly dispersed. These composites are simple to manufacture in large quantities, typically at low cost.

Orthotropic or transversely isotropic behavior can be induced in a material by inducing structural organization, such as 1-3 connectivity (FIG 1B). There are several methods of creating 1-3 composites, one example includes wafering a solid material into rod-like 108 structures, and backfilling the voids with the intended material. Others entail weaving fibers 108 through a semi-porous matrix or manually aligning long fibers 108 and then filling the surrounding area with the composite matrix 104. These techniques result in the structures 108 forming continuous columns that span the thickness of the composite.

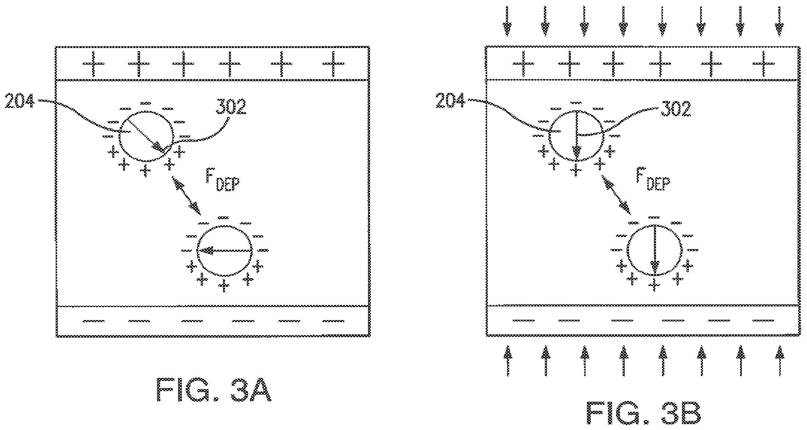

While there can be large increases in composite properties by utilizing 1-3 composites, they typically entail 'brute force' manufacturing techniques, and can be quite costly, labor intensive, and time consuming to produce. Nonuniform electric fields can be used to structure particles via the dielectrophoretic (DEP) force. The DEP force is based upon the surface charges induced on dielectric particles in an electric field, and the interactions between the polarized particles and the applied electric fields (FIG 3A). Structured 1-3 composites are created by utilizing the DEP force while the matrix material is still fluid. While the inclusions are still mobile, the DEP force structures them into column-like structures, where they are held until the composite matrix has solidified. Once completed, this technique successfully creates 1-3 structured composites with manufacturing techniques similar to those for 0-3 materials.

As described herein, methods are provided that utilize the piezoelectric nature of particles to generate composites with 1-3 connectivity. This is suitably carried out by utilizing piezoelectrophoresis (PEP). The PEP force is analogous to the DEP force, however, utilization of PEP is accompanied by the added benefit of obviating the need to pole the specimens prior to use (FIG 3B). By eliminating the need to apply a large electric field to the sample to induce net piezoelectricity, this technique allows the creation of large scale piezoelectric materials. Furthermore, it allows the use of new matrix materials, previously infeasible due to low dielectric strengths.

Though not wishing to be bound by theory, piezoelectrophoresis (PEP) relies upon the assertion that the application of hydrostatic pressure (or temperature change) to a piezoelectric particles (e.g., a sphere, fiber, rod, etc.) generates an electric potential equivalent to the induced potential of a dielectric particle in an electric field. This results in the ability of piezoelectric particles to experience an interparticle force analogous to the DEP force, but in the absence of an externally applied electric field. This PEP force is instead attributed to a stimulus that results in the generation of charge on the particle, such as hydrostatic pressure. As a note, any other stimulus that generates charge on a piezoelectric element is capable of producing this effect (e.g. temporally variant temperature via the pyroelectric effect (heating or cooling), sonication, application of x-ray energy, etc.).

An externally applied electric field that is applied at the same frequency with a cyclic hydrostatic pressure can result in the creation of "chains" and 1-3 structured composites. This field can be applied in phase or out of phase, depending on the materials utilized. Also, as the PEP torque causes the particles to align their net moment with the electric field, this can also result in the formation of net piezoelectric 1-3 structured composites without the need for an externally applied electric field during a standard poling procedure. If a cyclic hydrostatic pressure is applied without the addition of an external electric field, composites with 3-3 connectivity can also be created, exhibiting an increase in dielectric, piezoelectric, and mechanical properties compared to 0-3 composites.

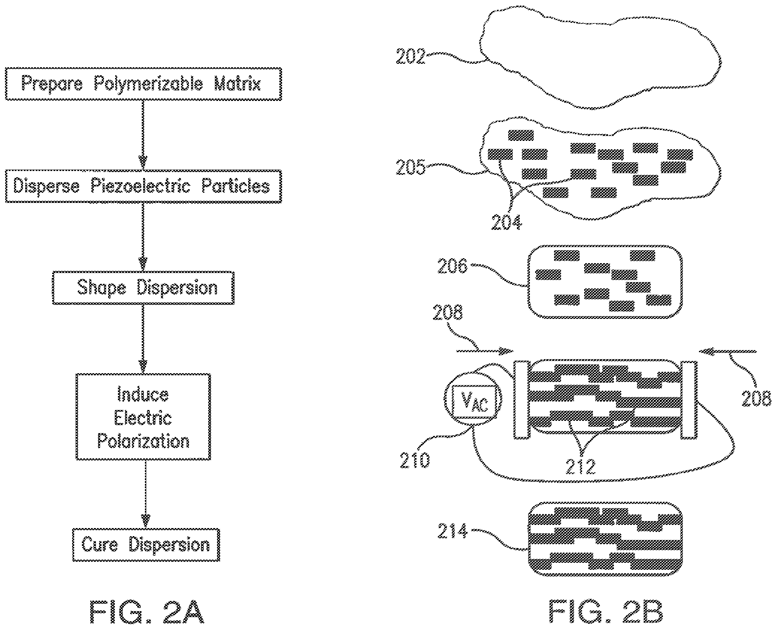

As described in FIGs 2A and 2B, in embodiments, methods of making a piezoelectric composite are provided. As used herein, a "composite" means a material comprising two or more components mixed or dispersed together. As used herein, a "piezoelectric" is a material that is capable of generating a voltage when a mechanical force is applied to the material.

The methods described herein suitably comprise preparing a polymerizable matrix 202. As used herein, "a polymerizable matrix" means a composition comprising monomers, polymers (two or more repeating structural units) or mixtures of monomers and polymers, or copolymers that can form a homogeneous or heterogeneous bulk composition when polymerized.

A plurality of piezoelectric particles 204 is dispersed in the polymerizable matrix to generate a dispersion 205. As used herein, "plurality" refers to 2 or more, suitably 5 or more, 10 or more, 50 or more, 100 or more, 500 or more, 1000 or more, etc., of an item, for example piezoelectric particles. The piezoelectric particles are dispersed in the matrixes via any suitable method, including mixing, stirring, folding or otherwise integrating the piezoelectric particles in the matrix so as to generate a fairly uniform mixture of the particles in the matrix.

The dispersion 205 is then shaped 206. As used herein, "shaped" or "shaping" refers to a mechanical or physical process by which a matrix (or dispersion) is changed to a desired form. "Shaping" can also include simply placing a matrix into a desired container or receptacle, thereby providing it with a maintained shape or form. It should be noted that the shaped form is not necessarily the final form, as additional processing (e.g., machining, forming, etc.) can be completed on the final, cured composite (see below). The act of shaping the dispersion for use in the methods described herein is primarily to give some initial structure to the dispersion prior to further processing. A rigid or specific shape is not required.

An electric polarization 302 (*see* FIG. 3A and 3B) is then induced in the piezoelectric particles 204 in the shaped dispersion. Suitably, at least 40% of the piezoelectric particles 204 form chains 212 as a result of the induction of the electric polarization. As used herein "chain" means 5 (five) or more piezoelectric particles connected to one another in a linear or semi-linear manner, i.e., piezoelectric particles at the ends of a chain are not connected to other piezoelectric particles in the same chain so as to form a loop. As used herein "columns" of piezoelectric particles are suitably formed by the stacking or aligning of more than one chain.

The dispersion is then cured to create a piezoelectric composite 214. The induction of the electric polarization is suitably maintained in the shaped dispersion until the matrix is fully cured, so as to keep the chain formation until the matrix is solidified.

As used herein "connected" or "connectivity" when referring to piezoelectric particles, means that the particles are within about 25% of a particle radius of one another. Suitably, the radius of the largest particle of the population of piezoelectric particles is used in determining if particles are connected. As used herein "radius" refers to the smallest particle aspect, and is not meant to be limited only to spherical particles, but is also applicable to fibers, rods, and other particle shapes. Connectivity between the particles is used to differentiate the situation where an electric polarization is created in the particles, but particles do not come to within about 25% of a particle radius of one another in a matrix material, but instead, remain dispersed within the matrix.

Chain formation requires connectivity or connection between particles in order to form the particles into chains. In embodiments, connected particles are within at least about 25%, at least about 20%, at least about 15%, at least about 10%, at least about 5% or at least about 1% of a particle radius of one another.

In embodiments, at least about 40% of the piezoelectric particles 204 form chains 212 as a result of the induction of the electric polarization, more suitably at least about 50%, at least about 55%, at least about 60% at least about 65%, at least about 70%, at least about 75%, at least about 80%, at least about 85%, at least about 90%, or at least about 95% of the piezoelectric particles 204 form chains 212 as a result of the induction of the electric polarization.

Suitably, the chains are aligned with one another. As used herein "aligned" is used to mean that the chains comprising the piezoelectric particles are oriented to within about ±10 degrees of each other. In embodiments, the chains comprising the piezoelectric particles are oriented to within about ±20 degrees of each other, more suitably to with within about ±15 degrees of each other, or within about ±10 degrees of each other or within about ±5 degrees of each other.

In exemplary embodiments, the monomers and/or polymers or copolymers of the polymerizable matrix 202 comprise a thermoset polymer, copolymer and/or monomer, a thermoplastic polymer, copolymer and/or monomer; or a thermoset/thermoplastic polymer or copolymer blend. Exemplary thermoset and thermoplastic polymers, copolymers and monomers are well known in the art, and include for example, polymers, copolymers and monomers of poly(vinylidene difluoride) (PVDF), poly(urethane), various epoxies (e.g., EPO-TEK® 302-3M; EPOXY TECHNOLOGY, INC, Billerica, MA), poly(ethylene), polystyrene), poly(methyl methacrylate) (PMMA), poly(ether ether ketone) (PEEK), poly(aryletherkietone) (PAEK), etc.

Suitably, piezoelectric particles 204 for use in the composites described herein exhibit a Perovskite crystalline structure, i.e., the same type of crystal structure as calcium titanium oxide ($CaTiO_3$). In embodiments, suitable piezoelectric particles include but are not limited to, particles of barium titanate, particles of hydroxyapatite, particles of apatite, particles of lithium sulfate monohydrate, particles of sodium potassium niobate, particles of quartz, particles of lead zirconium titanate (PZT), particles of tartaric acid and polyvinylidene difluoride fibers. Other piezoelectric particles known in the art can also be used in the composites described herein. Suitably, a single type of piezoelectric particle is used in the composites and methods of making the composites, though in other embodiments, mixtures of different types or classes of piezoelectric particles can also be used. In embodiments, the piezoelectric particles are on the order of less than about 1000 $\mu$m in size, suitably less than about 750 $\mu$m in size, suitably less than about 500 $\mu$m in size, suitably less than about 100 $\mu$m in size, less than about 10 $\mu$m, less than about 1 $\mu$m, less than about 500 nm, or less than about 100 nm in size. As used herein "particle" includes any shape or configuration of material, including spheres, fibers, angular shapes, rods, pieces or fragments of materials, flakes, shavings, chips, etc.

Exemplary methods of shaping 206 the dispersions comprising the piezoelectric particles and polymerizable matrix include, but are not limited to, injection molding, extrusion, compression molding, blow molding or thermoforming. Other suitable shaping methods can also be used. In other embodiments, the dispersion can simply be placed in a suitable container or other receptacle to hold the dispersion while the various other steps of the methods described herein are carried out.

In embodiments, inducing an electric polarization may comprise applying an electric field 210 in a direction to the shaped dispersion. Suitably, the electric field is applied in the direction (or perpendicular to the direction to account for negative dielectrophoresis) in which it is desired that resulting chains are to align. As shown in FIG. 3A, application of an electric field results in the induction of an electric polarization 302 in the particles. This effect is classically known as dielectrophoresis (DEP) as described herein.

Suitably, the electric field applied in such embodiments has a frequency of about 1 kHz to about 10 kHz and field strength of about 1 Volt/mm to about lkVolt/mm. For example, for DEP, an electric field having a frequency about 1 kHz to about 2 kHz, about 2 kHz to about 3 kHz, about 3 kHz to about 4 kHz, about 4 kHz to about 5 kHz, about 5 kHz to about 6 kHz, about 6kHz to about 7 kHz, about 7 kHz to about 8 kHz, about 8 kHz to about 9 kHz, about 9 kHz to about 10 kHz, or any other range or value within these ranges can be utilized. In embodiments, such electric fields will have a field strength of about 1 Volt/mm to about 500 Volt/mm, about 50 Volt/mm to about 500 Volt/mm, about 100 Volt/mm to about 500 Volt/mm, about 100 Volt/mm to about 400 Volt/mm, about 100 Volt/mm to about 300 Volt/mm, or about 200 Volt/mm to about 300 Volt/mm, as well as any range or value within these ranges. Suitably, the clectric field is applied as a sine wave having the characteristics described herein, though other wave shapes, including square waves, can be used.

In further embodiments, inducing an electric polarization suitably comprises applying a hydrostatic pressure 208 to the shaped dispersion or can comprise changing the temperature of the shaped dispersion, resulting piezoelectrophoresis. As demonstrated in FIG. 3B, piezoelectrophoresis (PEP) suitably results in both the formation of chains 212 of piezoelectric particles, while also alignment of dipoles 302 of the particles.

Application of hydrostatic pressure 208 suitably comprises application of a sine wave of about 344.7 kPa or 50 pounds per square inch (psi, where 1 psi = 6.895 kPa) to about 34470kPa or 5000 psi with a frequency of about 0.1 Hz to about 200 GHz. In exemplary embodiments, the sine wave can have a pressure of about 68.95kPa or 10 psi to about 13790kPa or 2000 psi, or about 689.5kPa or 100 psi to about 6895kPa or 1000 psi, or about 3447kPa or 500 psi to about 6895kPa or 1000 psi, or about 3447kPa or 500 psi, about 4137kPa or 600 psi, about 4826kPA or 700 psi, about 5516kPa or 800 psi, about 6205kPa or 900 psi or about 6895kPa or 1000 psi. Suitable frequencies include about 1 Hz to about 200 GHz, about 1 Hz to about 100 GHz, about 1 Hz to about 1 GHz, about 1 Hz to about 500 MHz, about 1 Hz to about 100 MHz, about 1 Hz to about 1 MHz, about 1 Hz to about 500 Hz, about 1 Hz to about 50 Hz, about 1 Hz to about 40 Hz, about 1 Hz to about 20 Hz, about 1 Hz, about 2 Hz, about 3 Hz, about 4 Hz, about 5 Hz, about 6 Hz, about 7 Hz, about 8 Hz, about 9 Hz, about 10 Hz, about 11 Hz, about 12 Hz, about 13 Hz, about 14 Hz, about 15 Hz, about 16 Hz, about 17 Hz, about 18 Hz, about 19 Hz or about 20 Hz. Other wave shapes, including square waves having the characteristics noted above, can also be used.

As discussed herein, by eliminating the need to apply a large electric field (e.g., on the order of 10 kV/mm or larger) to the sample to induce net piezoelectricity and "pole" the material, the methods described throughout allow for the production of materials having larger sizes and more freedom in shape and morphology of the final product thereby enabling more diverse uses for the composites. Thus, in embodiments, the induction of an electric polarization suitably does not include the application of an electric field.

In additional embodiments, though, an electric field can be applied in a direction to the shaped dispersion in combination with the application of the hydrostatic pressure or the change in temperature to induce the electric polarization. In such embodiments, a low-level electric field can help to further align the chains that form as a result of the PEP. In embodiments, this low level electric field can be applied with a frequency of about 1 Hz to about 100 Hz and a field strength of about 1 Volt/mm to about 1 kVolt/mm. For example, an electric field having a frequency about 1 Hz to about 75 Hz, about 1 Hz to about 50 Hz, about 1 Hz to about 40 Hz, about 1 Hz to about 30 Hz, about 1 Hz to about 20 Hz, about 1 Hz to about 10 kHz, about 1 Hz, about Hz, about 3 Hz, about 4 Hz, about 5 Hz, about 6 Hz, about 7 Hz, about 8 Hz, about 9 Hz, about 10 Hz, about 11 Hz, about 12 Hz, about 13 Hz, about 14 Hz, about 15 Hz, about 16 Hz, about 17 Hz, about 18 Hz, about 19 Hz, about 20 Hz, or any other range or value within these ranges can be utilized. In embodiments, such electric fields will have a field strength of about 1 Volt/mm to about 1000 Volt/mm, about 1 Volt/mm to about 500 Volt/mm, about 50 Volt/mm to about 500 Volt/mm, about 100 Volt/mm to about 500 Volt/mm, about 100 Volt/mm to about 400 Volt/mm, about 100 Volt/mm to about 300 Volt/mm, or about 200 Volt/mm to about 300 Volt/mm, as well as any range or value within these ranges.

In suitable embodiments, when the methods comprise inducing a electric polarization via the application of a hydrostatic pressure or a change in temperature, as well as the application of an electric field, the induction of the electric polarization and the application of the electric field can occur in any manner. For example, the induction of the electric polarization can occur before applying an electric field or the induction of the electric polarization can occur after the application of the electric field, In further embodiments, the induction of the electric polarization and the application of the electric field suitably occur simultaneously, i.e., the application of the hydrostatic pressure or temperature change occurs at the same time as the application of the electric field, for example both take place together or within seconds or minutes of each other.

In suitable embodiments, an electric field is applied at the same frequency with a cyclic hydrostatic pressure in the methods. The field can be in phase or out of phase with the hydrostatic pressure, depending on the materials utilized. When used in phase, the frequency of the electric field and the frequency of the hydrostatic pressure are applied so that the maximum amplitude of the cycle of each is reached at approximately the same time, thereby resulting in an in-phase application. The cycles can also be phase offset to account for losses in the particles and matrix that can cause a phase lag or gain between the polarization inducing stimulus and the polarization itself.

Exemplary methods of curing the polymerizable matrices so as to form the final composite are known in the art, and include, but are not limited to, cooling, UV curing, heat accelerated curing or compression curing of the dispersion.

In embodiments, the chains of piezoelectric particles produced in the composites prepared according to the methods described herein have a random orientation. However, in further embodiments, suitably at least about 10% of the chains are aligned to within about ±10 degrees of each other. In further embodiments, suitably at least about 10% of the chains are aligned to within about ±10 degrees of the direction of the applied electric field. This electric field can be either the electric field applied during DEP to form the chains, or the electric field applied during PEP to further align the chains.

More suitably at least about 20%, at least about 30%, at least about 40%, at least about 50%, at least about 60%, at least about 70%, at least about 80%, or at least about 90% of the chains are aligned to within about ±10 degrees of each other and/or of the direction of the applied electric field. More suitably, the chains are aligned to within about ±5 degrees of each other, or suitably with about ±5 degrees of the direction of each other and/or the applied electric field.

It is well within the level of those skilled in the art to determine the direction of chain alignment and its orientation with respect to an applied electric field.

In further embodiments, methods of making a tissue-stimulating piezoelectric composite are provided. As used herein, a "tissue-stimulating" composite as described throughout is suitably implanted or otherwise introduced into a patient so as to provide electric stimulation to a tissue of a patient when the composite is placed under any stress or strain, including transverse shear, bending, torsion, twisting, compression or tension. Exemplary tissues include, but are not limited to, bone, muscle, cartilage, tendons and organs (e.g., brain, heart, lungs). Suitably, the patients are mammals, including humans, dogs, cats, mice, rats, monkeys, etc.

In embodiments, the tissue-stimulating piezoelectric composites are bone-stimulating composites, including spinal implants for spinal fusion. The electric stimulation produced by the composites aids in stimulation of bone growth and osseointegration of the composite.

In suitable embodiments, tissue-stimulating piezoelectric composites are prepared by preparing a thermoset, thermoplastic, thermoset/thermoplastic or copolymer polymerizable matrix. A plurality of piezoelectric particles is dispersed in the polymerizable matrix to generate a dispersion. The dispersion is then shaped.

An electric polarization is induced in the piezoelectric particles in the shaped dispersion, wherein at least 40% of the piezoelectric particles form chains as a result of the induction of the electric polarization. The dispersion is cured to form the composite.

Exemplary methods of shaping the dispersion are described herein or otherwise known in the art, and include injection molding, extrusion, compression molding, blow molding or thermoforming.

Exemplary piezoelectric particles include particles exhibiting a Perovskite crystalline structure. Suitable particles include particles of barium titanate, particles of hydroxyapatite, particles of apatite, particles of lithium sulfate monohydrate, particles of sodium potassium niobate, particles of quartz, particles of lead zirconium titanate (PZT), particles of tartaric acid and poly(vinylidene difluoride) fibers.

As described herein, in suitable embodiments, an electric polarization is induced by applying an electric field in a direction to the shaped dispersion. In additional embodiments, an electric polarization is induced by applying a hydrostatic pressure to the shaped dispersion or changing the temperature of the shaped dispersion. In such embodiments, an electric field can also be applied in a direction to the shaped dispersion. Exemplary frequencies and field strengths of the electric fields for use in the methods are described throughout.

As described herein, in suitable embodiments, when the methods comprise inducing an electric polarization via the application of a hydrostatic pressure or a change in temperature, as well as the application of an electric field, the order of the induction of the electric polarization and the application of the electric field can occur in any manner. For example, the induction of the electric polarization can occur before the applying an electric field, or the induction of the electric polarization can occur after the application of the electric field. In further embodiments, the induction of the electric polarization and the application of the electric field suitably occur simultaneously, i.e., the application of the hydrostatic pressure or temperature change occurs at the same time as the application of the electric field, for example both take place together or within seconds or minutes of each other. Suitably, the electric field is applied at the same frequency with a cyclic hydrostatic pressure. The field can be in phase or out of phase with the pressure depending on the type of materials utilized.

Exemplary methods of curing the polymerizable matrix so as to form the final composite are known in the art, and include, but are not limited to, cooling, UV curing, heat accelerated curing or compression curing of the dispersion.

In embodiments, the chains of piezoelectric particles produced in the composites prepared according to the methods described herein have a random orientation. However, in further embodiments, suitably at least about 10% of the chains are aligned to within about ±10 degrees of each other. In further embodiments, suitably at least about 10% of the chains are aligned to within about ±10 degrees of the direction of the applied electric field. This electric field can be either the electric field applied during DEP to form the chains, or the electric field applied during PEP to further align the chains.

In embodiments, piezoelectric composites prepared by the methods described throughout are also provided. Also provided are tissue-stimulating piezoelectric composites prepared by the methods described herein.

In exemplary embodiments, piezoelectric composites comprising a polymer matrix and a plurality of piezoelectric particles are provided. Suitably, in the composites, at least 40% of the piezoelectric particles are in the form of chains. In embodiments, the composites have at least one dimension of 0.5 mm or greater, suitably at least one dimension of 1 mm or greater, or at least 5 mm or greater.

While in embodiments, the chains that are present in the composites have a random orientation, suitably at least about 10% of the chains are aligned to within ±10 degrees of each other.

More suitably at least about 20%, at least about 30%, at least about 40%, at least about 50%, at least about 60%, at least about 70%, at least about 80%, or at least about 90% of the chains are aligned to within about ±10 degrees of each other. More suitably, the chains are aligned to within about ±5 degrees of each other.

Suitably, the composites provided herein and prepared by the disclosed methods are 1-3 composites, for example as illustrated in FIG. 1B.

As described herein, suitably the polymer is a thermoset polymer, a thermoplastic polymer or a thermoset/thermoplastic polymer or copolymer blend. Exemplary polymers are described herein or otherwise known in the art. Suitably, the polymer is a PVDF polymer.

As described throughout, exemplary piezoelectric particles for use in the compositions exhibit a Perovskite crystalline structure. Suitable piezoelectric particles include, but are not limited to, particles of barium titanate, particles of hydroxyapatite, particles of apatite, particles of lithium sulfate monohydrate, particles of sodium potassium niobate, particles of quartz, particles of lead zirconium titanate (PZT), particles of tartaric acid and poly(vinylidene difluoride) fibers.

Suitably, when stressed or strained, including transverse shear, bending, torsion, twisting, compression or tension, the composites described herein generate a current density of between about 1 microamps/cm$^2$ to about 1 amp/cm$^2$. More suitably the composites generate a current density between about 1 microamps/cm$^2$ to about 500 microamps/cm$^2$, about 1 microamps/cm$^2$ to about 400 microamps/cm$^2$, about 1 microamps/cm$^2$ to about 300 microamps/cm$^2$, about 1 microamps/cm$^2$ to about 250 microamps/cm$^2$, about 1 microamps/cm$^2$ to about 200 microamps/cm$^2$, about 1 microamps/cm$^2$ to about 150 microamps/cm$^2$, about 1 microamps/cm$^2$ to about 100 microamps/cm$^2$, about 1 microamps/cm$^2$ to about 90 microamps/cm$^2$, about 1 microamps/cm$^2$ to about 80 microamps/cm$^3$, about 1 microamps/cm$^2$ to about 70 microamps/cm$^2$, about 1 microamps/cm$^2$ to about 60 microamps/cm$^2$, about 1 microamps/cm$^2$ to about 50 microamps/cm$^2$, about 1 microamps/cm$^2$ to about 30 microamps/cm$^2$, about 10 microamps/cm$^2$, about 20 microamps/cm$^2$, about 30 microamps/cm$^2$, about 40 microamps/cm$^2$, about 50 microamps/cm$^2$, about 60 microamps/cm$^2$, about 70 microamps/cm$^2$, about 80 microamps/cm$^2$, about 90 microamps/cm$^2$, or about 100 microamps/cm$^2$. Suitably, the current density is a constant, direct current density and the electric potential of the composites are negative.

Suitably, the composites have at least one dimension of 0.5 mm or greater, suitably at least one dimension of 1 mm or greater, or at least one dimension of about 5 mm or greater, or at least one dimension of about 10 mm or greater, or about 20 mm or greater, about 30 mm or greater, about 40 mm or greater, about 50 mm or greater, about 60 mm or greater, about 70 mm or greater, about 80 mm or greater, about 90 mm or greater, or about 100 mm or greater. While any dimension and shape of composite can be generated using the methods described, an advantage of the methods provided herein is that composites having at least one dimension greater than about 0.5 mm or about 1 mm or about 10 mm can readily be generated, as compared to other methods of generating piezoelectric composites where the materials are limited in size.

Also provided are methods of preparing piezoelectric composites, including tissue-stimulating piezoelectric composites, such as spinal implants, that are made via physical alignment of fibers in a relatively non-conductive polymer matrix to form a 1-3 piezoelectric composite structure. Such 1-3 piezoelectric composites may have different characteristics as compared to structured 1-3 composite created using the DEP force, as described herein, in terms of toughness, fracture properties, and ease of manufacturing. Examples of suitable piezoelectric particles (i.e., fibers) are described herein, as are suitable polymeric matrices. Suitably, alignment of the fibers in these embodiments comprises physically moving the fibers into the desired position prior to curing or other manufacturing of the composite.

Also provided herein are tissue-stimulating piezoelectric composites. Suitable composites comprise a polymer matrix and a plurality of piezoelectric particles, wherein at least 40% of the piezoelectric particles are in the form of chains, and the composite is a 1-3 composite.

As described herein, at least about 10% of the chains are oriented within ±10 degrees of each other, more suitably at least about 50% of the chains are oriented within ±10 degrees of each other.

In embodiments, the piezoelectric particles exhibit a Perovskite crystalline structure. Suitable piezoelectric particles include but are not limited to particles of barium titanate, particles of hydroxyapatite, particles of apatite, particles of lithium sulfate monohydrate, particles of sodium potassium niobate, particles of quartz, particles of lead zirconium titanate (PZT), particles of tartaric acid and poly(vinylidene difluoride) fibers.

In suitable embodiments, the tissue-stimulating piezoelectric composites generate a current density of between about 1 microamp/cm$^2$ to about 250 microamps/cm$^2$ when compressed. This current density is ideally provided to increase tissue healing, e.g., the rate of bone fusion. As the piezoelectric composites described herein generate current density simply in response to pressure, no additional power source is required.

In further embodiments, the composites, including tissue-stimulating piezoelectric composites, are 1-3 composites. Suitably, the composites provided herein possess a piezoelectric charge coefficient $d_{33}$ of the composite between 1% and 100% of the bulk piezoelectric charge coefficient from which the composite is created.

Suitably, the composites provided herein possess a dielectric constant $\varepsilon_{33}$ of the composite between 1% and 100% of the bulk dielectric constant of the filler material from which the composite is created.

These ranges are functions of several variables that can be altered between composites. As the composite material approaches a 100% volume fraction of piezoelectric particles, the properties approach the values of the bulk piezoelectric material. In the embodiments, the volume fractions are often below 50%. Another key factor these properties depend on is the aspect ratio of the piezoelectric particles. If the aspect ratio is above 30, suitably above 100, then a composite with only a 30% volume fraction of fibers suitably possesses a $d_{33}$ close to 100% of the bulk material. If the aspect ratio is closer to 10, then for a 30% volume fraction, the $d_{33}$ would be much closer to 25% of the bulk material value.

This same justification holds for the dielectric constant, except that there is a fairly linear relationship between dielectric constant and volume fraction.

Suitably after curing, the piezoelectric composites are further shaped or molded into their desired final shape. In the case of tissue-stimulating composites, these final shapes will be determined by the final in-patient use, taking into account patient anatomy, size requirements and ultimate use.

In embodiments, the tissue-stimulating piezoelectric composites can further comprise a coating on their surface, e.g., a polymer coating or shell, to facilitate biocompatibility, or in some cases a coating to deliver a desirable compound or drug to the tissue. For example, a coating such as hydroxyapatite or other bone growth stimulant, drug, or resorbable scaffold polymers such as PLA (polylactic acid) or PLLA (poly-L-lactide) or PGA (polyglycolle acid) or antibiotics or nonresorbable coatings such as poly(ether ether ketone) (PEEK), poly(aryletherkeptone) (PEAK) or other suitable materials, can be coated on the composites.

In embodiments, the piezoelectric composites are provided with an insulator which may be made of ultra high weight polyethylene or titanium oxide or any other suitable non-conductive non-toxic biocompatible material. The insulator can be provided on the piezoelectric element but not where a tissue-interface is desired (i.e., contact with a tissue). The conductive material can be a commercially available biocompatible epoxy composition or it can be a thin layer of a precious metal such as gold or silver, or other metals such as titanium and its alloys, tantalum or cobalt chromium alloys.

Exemplary tissue-stimulating composites that can be produced according to the methods provided herein include bone plates, bone screws, bone implants, spinal implants, etc.

In embodiments, the tissue-stimulating composites described herein are strain coupled to bone or other body tissue so as to generate charge as the tissue undergoes strain, and the generated charge is applied via electrodes to a region where it is desired to stimulate a tissue, e.g., bone growth (*see, e.g.*, U.S. Patent No. 6,143,035. In embodiments, the composites described herein can be attached by pins or bone screws to a bone and the poles of the piezoelectric element are connected via leads to carry the charge remotely and couple the charge to promote healing.

Thus, the strains from the natural loading of the tissue (e.g. bone) are coupled into the piezoelectric composites and generate charge across the poles of that composite which creates a current flow.

In general, the direction of current flow created by the material will be alternating, dependent on whether the implant is being loaded, or unloaded (e.g., by the patient). Suitably, the direction of current flow is controlled through the use of a rectification circuit attached to the device prior to use, including implantation in a patient. Further circuitry can be involved to condition the signal, store and deliver excess energy, and power additional features or functions of the device (i.e. enabling telemonitoring, lab on a chip devices, etc.).

When an implant (e.g., a spinal implant) is loaded, it may generate a positive charge on top, and negative on the bottom. However, when unloaded, it will then generate a negative charge on top, and positive on the bottom. This implant will operate suitably under cyclic loading (i.e. walking), as such, an AC current would be created. In order to deliver DC stimulation, as that is the most effective form of electrical stimulation, the AC signal must be rectified before delivery to the patient.

In general, the direction of current flow induced in a patient is dependent on the pole orientation of the piezoelectric composite and the direction of strain loading, e.g., tensile or compressive strain, applied to the composite. Suitably, the direction of current flow is selected during manufacture and configuration of appropriate circuitry so that implantation of the composite produces the desired effect, e.g., enhanced bone growth effects.

In addition to use as tissue-stimulating composites, the piezoelectric composites described herein can suitably be used in any number of additional applications and configurations. Methods of shaping, forming or otherwise preparing the composites described herein to be utilized in such applications are well within the level of one of ordinary skill in the art of the various applications.

For example, piezoelectric composites described herein can be utilized in the following:

Carbon black impregnated PMMA or other polymers to generate a matrix of composites;
The composites can be filled into an expandable device to fill space to eliminate trial size implants in general;
The composites can be filled into any pressurized cavity that can be filled with bone cement and have a metal implant inserted, e.g., for bone stimulation;
Fracture fixation devices (bone plates, screws, pins on external fixators, etc.);

Dental implants for bone healing;

Posterior instrumentation for spine fusion (pedicle screws, rods, etc.);

Linkage to power a battery for a pacemaker;

Linkage to power any internal device/sensor;

Attachment to any load bearing part to stimulate internal organ/tissue healing;

Lab on a chip devices that need power supplies;

Telemetry powering for sensing ... "built in sensors";

Use in a continuous extrusion process for piezoelectric rods;

In combination with slight twisting/distortion/rotation of electrical field (or could be mechanical) during extrusion as rods/structures before curing to generate twist coupled sensors and actuators;

A variety of energy/power harvesting devices including:

Tires on any vehicle to power rechargeable batteries and provide vibrational damping;

Drive shaft on a car to power rechargeable batteries;

Car paint or part of a car grill to power rechargeable batteries;

"Rubber" surface on a floor to capture loads and convert to power;

Application on load-bearing structures in vibration generating devices in a household to feed to power grid;

Roads to capture vehicular loads;

More efficient wind mills - blades or other structures loaded to generate power;

Plates/structures in oceans/seas to capture wave loads;

Bleachers in sport stadiums to power novelty lights or feed power into the grid as a function of fan loading of bleachers;

Body of cell phone to recharge batteries;

Structured components in building for energy harvest/sensors/damping;

Shingles on houses to translate wind forces and feed to power grid;

Bridge components for sensing/power generation;

Parts in power tools like jackhammers or drills for energy harvest/sensors/damping;

Parts of construction equipment for energy harvest/sensors/damping;

Mechanical damping with piezoelectric structures;

Use in structures in regions of high seismic activity to capture early detection and damping;

Hook up to grid to form a giant network of sensors;

Snow ski vibration damping;

Self-heating boots;

Shoes with lighting;

Sensors for clothing;

Fabric made for various applications;

Sound proofing materials for damping;

Sails of sail boats to generate ship power;

Incorporate with any power plant system to increase efficiency;

Poles in power lines;

Piezoelectric transmission lines or conductive cables;

Road sensing lines/pads to trigger stop lights, sense presence of cars, etc;

Self-powered exoskeleton;

Electrorheological fluids;

Fluid brakes and clutches in vehicles that change viscosity based on applied pressure instead of electric field;

Heart blanket/sock for heart failure treatment (wrap around heart and contract based on applied voltage);

Treads on tanks;

Remote sensor with sustained power from loading/vibration;

Front fork on bike to power bike devices or provide vibration damping;

Total disc replacement. Endplates constructed of piezoelectric composite, with negative electrodes lining the interface between device and vertebrae, while the positive terminal is placed near the center of the device to ensure bone does not grow into, and impinge the dynamic parts. This should improve the bond between the device and vertebrae, while strengthening the vertebrae to avoid endplate subsidence, and ideally further securing the device so it does not migrate;

Use of composites in a positive (healing of tissue) or negative (stopping tissue growth) in any novel implant;

Reduction of biofouling in implanted sensors through generation of surface charges;

Air filters to kill bacteria/other pathogens through surface charges; and

Self-sanitizing surface/structures.

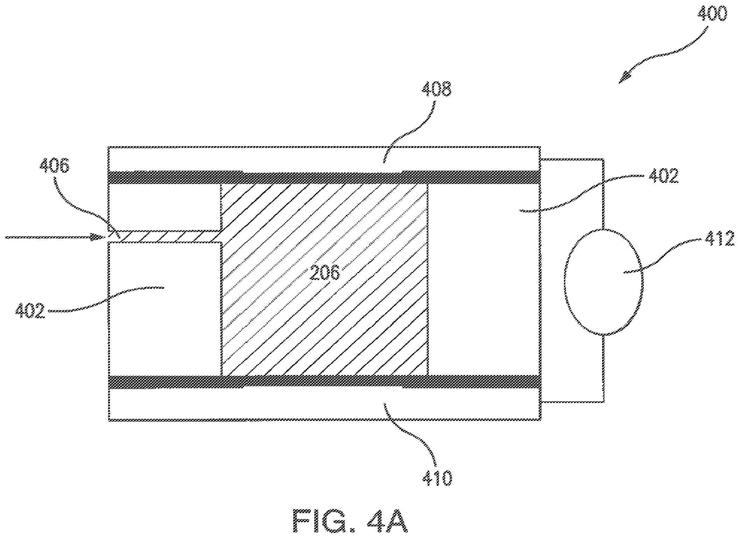

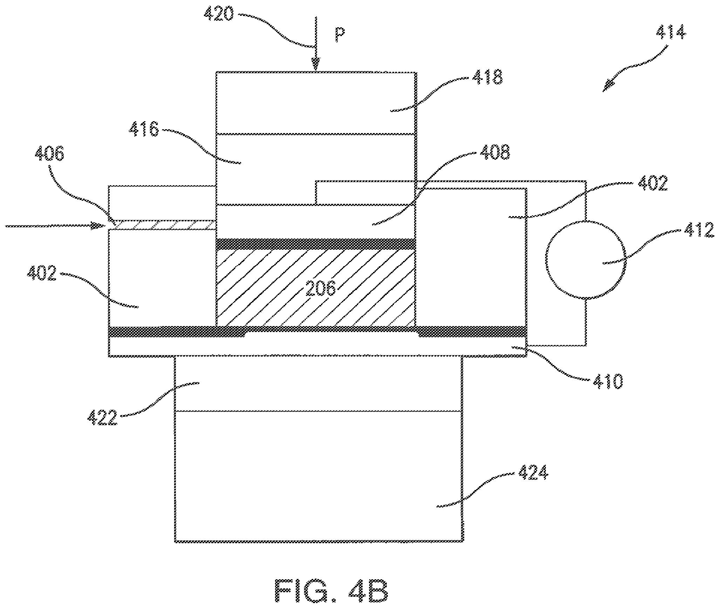

FIG. 4A shows an exemplary manufacturing set-up for use with dielectrophoretic (DEP) formation of piezoelectric composites described herein. FIG. 4B shows an exemplary manufacturing set-up for use with piezoelectrophoretic (PEP) formation of piezoelectric composites described herein.

The DEP manufacturing set-up 400 shown in FIG. 4A suitably comprises a mold apparatus 402 for holding shaped dispersion 206. Mold apparatus 402 suitably comprises an inlet 406 for introduction of dispersion 205 comprising the polymerizable matrix 202 material and dispersed piezoelectric particles 204. The set-up further suitably comprises an electric field generator 412, connected to a first electrode 408 and second electrode 410, for application of an electric field to the shaped dispersion.

The PEP manufacturing set-up 414 shown in FIG. 4B, is a modification of the DEP set-up 400. PEP set-up suitably further comprises insulating connectors 416 and 422, separating electrodes 408 and 410 from actuator 418 and load cell 424, respectively. Actuator 418 and load cell 424, are suitably a material testing system (MTS), e.g., an MTS 858 MiniBionix (MTS Systems Corporation, Eden Prairie, MN), comprising a plunger and load cell capable of applying a cyclic pressure 420 to the shaped dispersion 206. Inclusion of insulating connectors 416 and 422, allows for the application of an electric field as well as the hydrostatic pressure, as described herein. Through computer or other external control, a cyclic pressure can be generated at the same frequency with an applied electric field. The electric field and the cyclic pressure can be applied in phase or out of phase with one another depending on the types of materials utilized.

**Examples**

**Example 1: Preparation of 1-3 Composite using DEP**

Structured 1-3 composites were prepared using dielectrophoresis (DEP). The polymerizable matrix for these composites was a two part resin (302~3M, Epotek), and the particles were 5 micrometer barium titanate. Composites were structured using an electric field strength of 1 KV/mm and a frequency of 1 KHz. The resulting dielectric and piezoelectric properties of the composite materials were characterized using well-known techniques as described below.

Dielectric characterization was conducted using a Hioki 3522-50 LCR meter (Hioki EE Corporation, Negano, Japan). This meter is used to assess the capacitance, and resistance of the samples. This information, coupled with knowledge of sample geometry, can be used to determine a sample's resistivity, conductivity, and dielectric constant. The meter can also assess the dielectric loss factor ($\tan\partial$). Measurements are carried out at room temperature, at frequencies from DC to 1 KHz. Confidence intervals for each setup are constructed using a Student's T-test. Comparisons between control, DEP, and PEP structured materials at each volume fraction are conducted using one-way ANOVA.

Determination of piezoelectric properties are measured via direct or resonance methods. Resonance methods are widely used for piezoceramic crystals, and are highly accurate in that instance. However, when mechanical losses are high, which is likely in a quasi 1-3 composite, the quality of the results degrades. The direct method is easily implemented utilizing a material testing system (MTS), and can readily provide accurate results for material use at low frequencies. Stress is applied to the sample, while simultaneously recording the charge generated by the sample. This is done by placing a capacitor in electrical parallel with the sample, and recording the voltage. Since capacitors follow the relation: $Q = C^*V$, a plot of charge vs force can be generated. The slope of this line represents the piezoelectric charge coefficient The principal charge coefficient ($d_{33}$, $d_{32}$, and $d_{31}$) is also measured. Electrodes are applied to samples in the 3 direction during manufacture, and as such, $d_{33}$, $d_{32}$, and $d_{31}$, can be readily measured. The piezoelectric voltage coefficients ($g_{ij}$) can be calculated based on the d-coefficients, and dielectric constant of the material. This occurs as $g_{ij} = d_{ij}/\varepsilon$. The piezoelectric properties provide information key to the material's use as both sensing and actuating elements. Confidence intervals for each setup are constructed using a Student's T-test. Comparisons between control, DEP, and PEP structured materials at each volume fraction are conducted using one-way ANOVA.

Results obtained compare well to the models presented for 1-3 composites in Equations 3 and 4, below, as shown in FIG. 4.

$$d_{33_{0-3}} = \left(\frac{n\,\Psi\varepsilon_{0-3}}{n\varepsilon_{0-3}+(\varepsilon_2-\varepsilon_{0-3})}\right)d_{33_2} \quad (Eq3) \qquad d_{33_{1-3}} = \left(\frac{\Psi(1+R^2)\varepsilon_1 Y_{33_2}}{(\varepsilon_2+R\varepsilon_1)((1+R\Psi)Y_{33_2}+(1-\Psi)RY_1)}\right)d_{33_2} \quad (Eq4)$$

Piezoelectric charge coefficient for a 0-3 composite
- $\varepsilon_{0-3}$ – Dielectric constant of the 0-3 composite
- $\varepsilon_2$ – Dielectric constant of the particles
- $d_{33_2}$ – Piezoelectric particle charge constant
- n – Inverse depolarization factor
- $\Psi$ – Particle volume fraction

Piezoelectric charge coefficient for a 1-3 composite
- $\varepsilon_1$ – Dielectric constant of the matrix
- $\varepsilon_2$ – Dielectric constant of the particles
- R– Ratio of particle size to interparticle spacing
- $\Psi$ – Particle volume fraction
- $Y_1$ – Modulus of elasticity of the matrix
- $Y_{33_2}$ – Modulus of elasticity of the particles in the poled direction
- $d_{33_3}$ – Piezoelectric particle charge constant

**Example 2: Piezoelectric Composite Spinal Fusion Interbody Implant**

Provided herein is the development of a piezoelectric composite biomaterial and interbody device (spinal implant) design for the generation of clinically relevant levels of electrical stimulation to help improve the rate of fusion for in patients.

A lumped parameter model of the piezoelectric composite implant was developed based on a model that has been utilized to successfully predict power generation for piezoceramics. Seven variables (fiber material, matrix material, fiber volume fraction, fiber aspect ratio, implant cross-sectional area, implant thickness, and electrical load resistance) were parametrically analyzed to determine their effects on power generation within implant constraints. Influences of implant geometry and fiber aspect ratio were independent of material parameters. For a cyclic force of constant magnitude, implant thickness was directly and cross-sectional area inversely proportional to power generation potential. Fiber aspect ratios above 30 yielded maximum power generation potential while volume fractions above 15 percent showed superior performance. These results demonstrate the feasibility of using composite piezoelectric biomaterials in medical implants, such as spinal implants, to generate therapeutic levels of direct current electrical stimulation.

Methods

Model

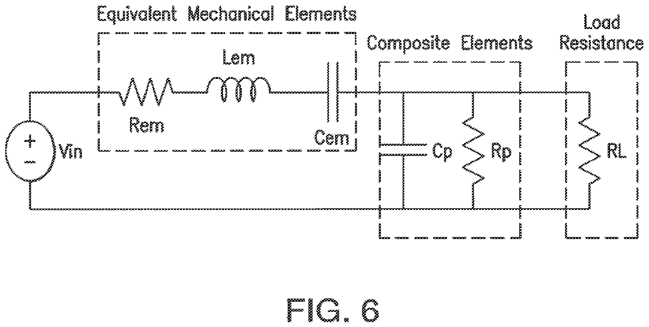

A model was developed to predict the power output of piezoelectric composites (16). The piezoelectric composite model was developed based on a similar model that has been utilized to successfully predict power generation for piezoceramics (17, 18). This circuit model can be broken down into four different sections: input voltage, equivalent mechanical elements, composite impedance, and load resistance (FIG. 6). The load resistance (RL) is the electrical resistance of the object to which the electrical power is being delivered.

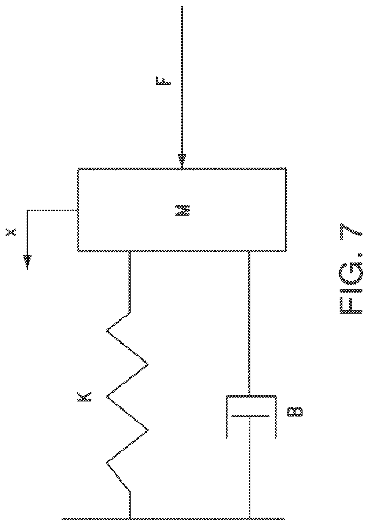

The circuit model was developed by constructing a lumped parameter model of the mechanical system, based on the mass, damping, and stiffness of the composite (FIG. 7).

This well-known model can be described by the second order differential equation presented in Equation 5.

$$F = M\ddot{x} + B\dot{x} + Kx \qquad \text{(Eq. 5)}$$

F     = external force
M    = effective mass
B     = damping
K     = stiffness
x     = mass displacement
$\dot{x}$     = mass velocity
$\ddot{x}$     = mass acceleration

This model is coupled to the circuit model shown in FIG. 6 through the use of a piezoelectric transformer ratio, φ (18). When used, this ratio affects the mechanical performance described in Equation 5 by introducing an electrical damping term that effectively describes the amount of energy transferred from the mechanical to electrical system (19).

$$\phi = \frac{st}{Ad} \qquad \text{(Eq. 6)}$$

t     = Thickness
A    = Cross-sectional area
s     = Composite compliance

When applied to the mechanical elements of the spring mass damper system, the transformer ratio establishes equivalent circuit elements which describe the conversion of mechanical vibrations to electrical energy (Equations 7-10).

$$Rem = \phi^2 B \qquad \text{(Eq. 7)}$$

$$Lem = \phi^2 M \qquad (Eq. 8)$$

$$Cem = (\phi^2 K)^{-1} \qquad (Eq. 9)$$

$$Vin = \phi F \qquad (Eq. 10)$$

Furthermore, the composite implant's electrical impedance can be determined by Equations 11 and 12. While piezoceramics are primarily capacitive, piezoelectric composites also include a polymer matrix that acts predominantly as a resistor at low frequencies. These were placed in parallel, representing the two parallel paths electricity has through the composite: through the capacitive fibers or the resistive matrix. Combined, these elements represent the electrical impedance of the composite material.

$$Cp = \left(1 - \frac{d^2}{s\varepsilon}\right)\frac{\varepsilon A}{t} \quad (Eq. 11)$$

$$Rp = \frac{\rho_C t}{A} \quad (Eq. 12)$$

The equations presented above have been utilized and validated for low frequency homogeneous piezoceramic materials (18). However, in order to utilize them to analyze piezoelectric composite materials, several composite material properties must first be defined. The composite's dielectric constant and piezoelectric charge coefficient have been theoretically and experimentally determined to follow Equations 13 and 14 for high aspect ratio fibers (12).

$$\varepsilon = \Psi\left(\frac{\left(\Gamma_{eff}\varepsilon_2 - \varepsilon_1\right)\varepsilon_2}{(\varepsilon_2 - \varepsilon_1)} - \left(\Gamma_{eff}d_{33}\right)^2 \frac{1-\Psi}{\Psi s_1 + (1-\Psi)s_2}\right) + (1-\Psi)\varepsilon_1$$

$$(Eq. 13)$$

$$d = \left(\frac{\Psi s_1}{\Psi s_1 + (1-\Psi)s_2}\right)\Gamma_{eff}d_{33} \qquad (Eq. 14)$$

where

$\varepsilon$ = Composite dielectric constant
$\varepsilon_1$ = Matrix dielectric constant
$\varepsilon_2$ = Fiber dielectric constant
$s_1$ = Matrix compliance
$s_2$ = Fiber compliance
$d$ = Composite piezoelectric charge constant
$d_{33}$ = Fiber piezoelectric charge constant
$\Gamma_{eff}$ = Effective ratio of the electric field acting on the fiber
$\Psi$ = Fiber volume fraction

The composite material's elastic modulus and electrical resistivity are based on equations for composites materials (Equations 15, 16). For these equations, 0-3 composite equations were used to approximate the quasi-1-3 material, and produce a conservative estimate of electrical power generation (20).

$$E_c = E_1\left(1 + \frac{3\left(\frac{E_2}{E_1} - 1\right)\Psi}{\left(\frac{E_2}{E_1} + 2\right) - \left(\frac{E_2}{E_1} - 1\right)\Psi}\right) \qquad (Eq. 15)$$

where

$E_1$=Matrix elastic modulus
$E_2$=Fiber elastic modulus

$$\rho_C = \rho_1 \left( 1 + \frac{3\left(\frac{\rho_2}{\rho_1}-1\right)\psi}{\left(\frac{\rho_2}{\rho_1}+2\right)-\left(\frac{\rho_2}{\rho_1}-1\right)\psi} \right) \text{(Eq. 16)}$$

where

$\rho_1$ = Matrix electrical resistivity
$\rho_2$ = Fiber electrical resistivity

Environmental Variables

The performance of a piezoelectric power generator depends on multiple variables. These variables relate not only to the material composition and implant geometry, but the environmental operating conditions as well. The environmental operating conditions, which include the applied force, frequency of compression, and electrical resistance of the surrounding tissue, have been reported by other investigators, and are discussed below (15, 21-27).

The force on the implant is primarily controlled by the weight of the patient and the patient's activities. The majority of the patient's upper body weight is supported by the spine. Furthermore, after a lumbar fusion, the majority of this weight is transferred directly through the fusion cage. During common activities such as walking, the force on the intervertebral disc in the lumbar region can range from 1.0 to 2.95 times body weight (21-24). However, with the inclusion of posterior instrumentation, the force on the implant itself is halved (25). For patients that have just undergone a spinal fusion and are recovering from surgery, walking is one of the most intense activities that can be expected. Since the average weight for an adult is reported to be 608 N (26), the average person would load an implant with between 300 and 900 N while walking. For this model, an intermediate value of 500 N was utilized as the applied force.

The frequency of implant compression also depends on the intensity of the activities performed by the patient Most activities occur with frequencies less than 5 Hz. Walking, for example, usually occurs at a frequency between 1.2 and 2 Hz (23). It is possible to increase the frequency of implant stimulation by applying a high frequency, low amplitude stimulus to the patient, such as ultrasound; however, this would require additional patient compliance, and likely visits to the doctor or physical therapist. For this model, 1.2 Hz was used for the frequency of implant compression.

At the present time, FDA approved DC electrical stimulation devices are designed to deliver the appropriate current density to bone with an electrical impedance from 0 to 40 k$\Omega$ (15). Experimentally, bone undergoing fracture healing has reported impedances between 4 and 7 k$\Omega$ (27). The load resistance that generates maximum power is determined in the subsequent analysis, and used to validate a stand-alone implant.

Material and Implant Geometry Variables

In addition to the environment conditions, several of the variables affecting power generation can be readily controlled during the manufacturing process. These include proper material selection and implant geometry. The materials used in the composite suitably comprise a polymer matrix embedded with a dispersion of aligned piezoelectric fibers. The polymer matrix provides structural stability for the brittle, ceramic fibers, while the fibers provide the net piezoelectric properties to the composite.

For this study, two different materials are investigated for the piezoelectric fibers: PZT and BaTiO$_3$ (Table 1).

Table 1: Material properties used in theoretical analysis.

| | Material | Elastic Modulus (GPa) | Dielectric Constant | Resistivity ($\Omega$*cm) | $d_{33}$ (pC/N) |
|---|---|---|---|---|---|
| Fiber Materials | PZT | 63 | 1350 | 1,0*1015 | 300 |
| | BaTiO$_3$ | 67 | 1000 | 1,0*1010 | 120 |

(continued)

| | Material | Elastic Modulus (GPa) | Dielectric Constant | Resistivity ($\Omega*cm$) | $d_{33}$ (pC/N) |
|---|---|---|---|---|---|
| Matrix Materials | Epotek 302-3M | 1.7 | 3.3 | 1.0*1013 | -- |
| | PEEK | 3.6 | 3.3 | 4.9*1016 | -- |
| | PVDF | 2 | 8.5 | 1.5*1014 | -- |
| | PVDF-TrFE-CFE | 0.5 | 50 | 9,9*1013 | -- |

PZT is one of the most commonly used piezoelectric materials due to its high piezoelectric properties and coupling coefficient (28). $BaTiO_3$ is considered a viable option due to its biocompatibility and current use in implantable medical devices.

Several matrix materials are analyzed including epoxy, PEEK, PVDF, and PVDF-TrFE-CFE. PEEK is suitably utilized as it is commonly used in fusion cages due to its high strength, similar stiffness to bone, and excellent biocompatibility (29). A two-part epoxy (Epotek 302-3M) has been used previously in piezoelectric composite research. It was analyzed in the model to provide comparison to other composites (30). PVDF and PVDF-TrFE-CFE were analyzed due to their promising theoretical results with piezoelectric particle composites and biocompatibility (16). The material properties used in the theoretical model are shown in Table 1.

Additionally, implant geometry also affects the expected power generation. Cross-sectional area and thickness measurements were taken from commercially available small TLIF spinal fusion cages as well as large ALIF cages to provide a reasonable range of inputs for the theoretical model. Cross-sectional areas of these cages ranged from 120 to 325 mm$^2$ and the thickness ranged from 5 to 20 mm. Typical ranges of fiber volume fraction (0-40%) and aspect ratios (1-1000) were also analyzed. Since tissue electrical properties are variable, the influence of load resistance was also studied by varying the circuit variable from 0-10 T$\Omega$. Table 2 lists the ranges of these controllable variables that were analyzed to determine the influence of implant variables on power generation.

Table 2. Ranges of controllable variables for the composite when used as a spinal fusion cage.

| | Controllable Variables | Range |
|---|---|---|
| **Fiber Variables** | Volume Fraction | 0-4% |
| | Aspect Ratio | 1-1000 |
| **Implant Geometry** | Cross-sectional Area | 120-325 mm$^2$ |
| | Thickness | 5-20 mm |
| **Circuit Variable** | Load Resistance | 0-10 T$\Omega$ |

Results

Seven variables from the model were studied to determine their influence on the electricity generated by the composite: fiber material, matrix material, fiber volume fraction, fiber aspect ratio, implant cross-sectional area, implant thickness, and load resistance.

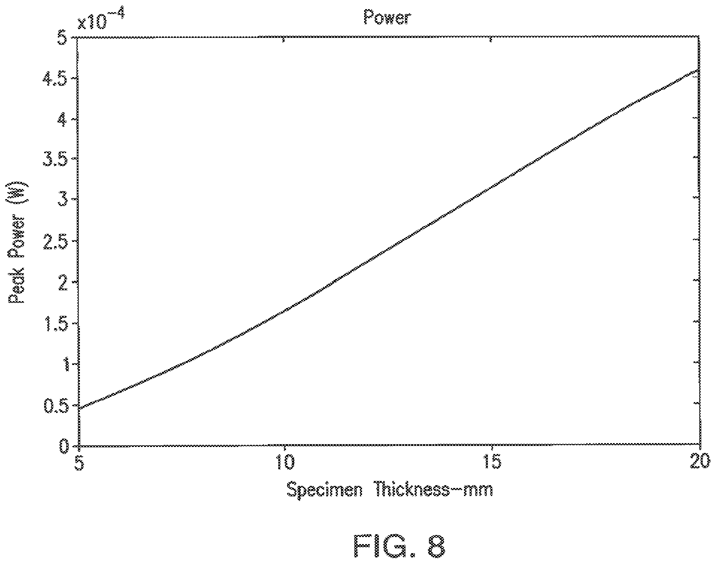

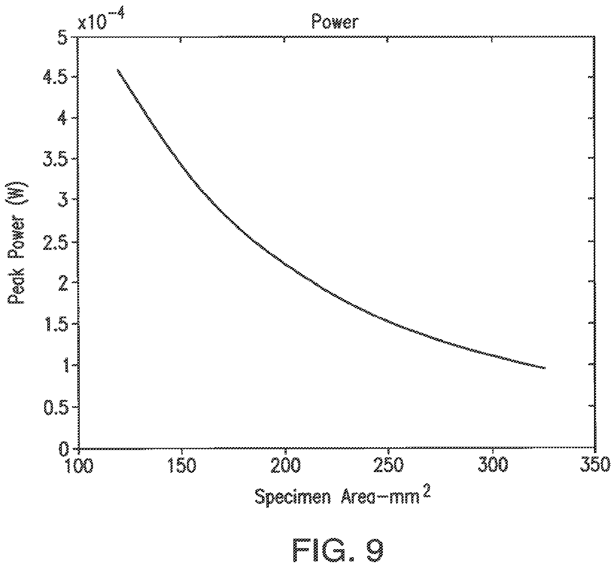

Preliminary tests established that for a cyclic force of constant magnitude, maximum power was generated with the largest implant thickness and the smallest cross-sectional area (FIGs. 8 and 9). These results were unaffected by changes in other variables. For the following analyses, the implant thickness was set to 20 mm and the cross-sectional area was set to 120 mm$^2$ in order to determine the potential maximum power output of this device.

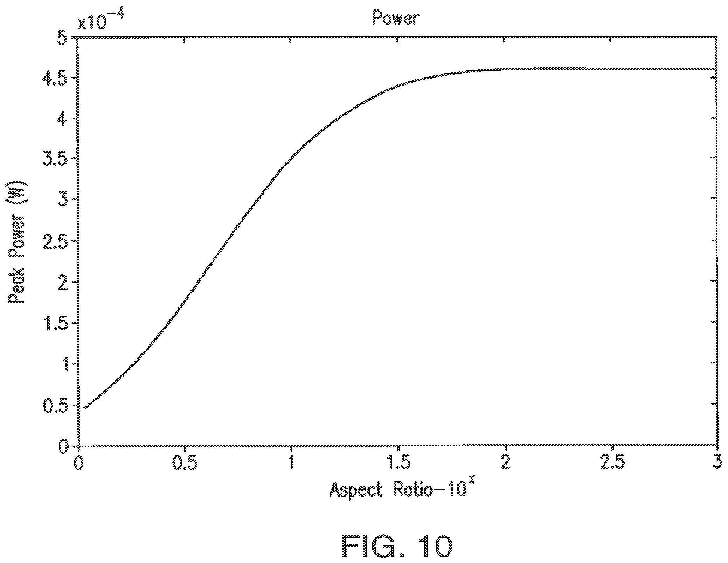

The influence of fiber aspect ratio shows a large increase in power output (880%) from ratios of 1-30, followed by a smaller increase of 7.0% for ratios between 30-100, and almost no change (<1%) from 100-1000 (FIG. 10). Therefore, in order to generate maximum power, the fibers used in the composite suitably have an aspect ratio of at least 30. These trends result from drastic increases in material properties associated with high aspect ratio particles and correspond well with the experimental results of Van den Ende *et al.* (12).

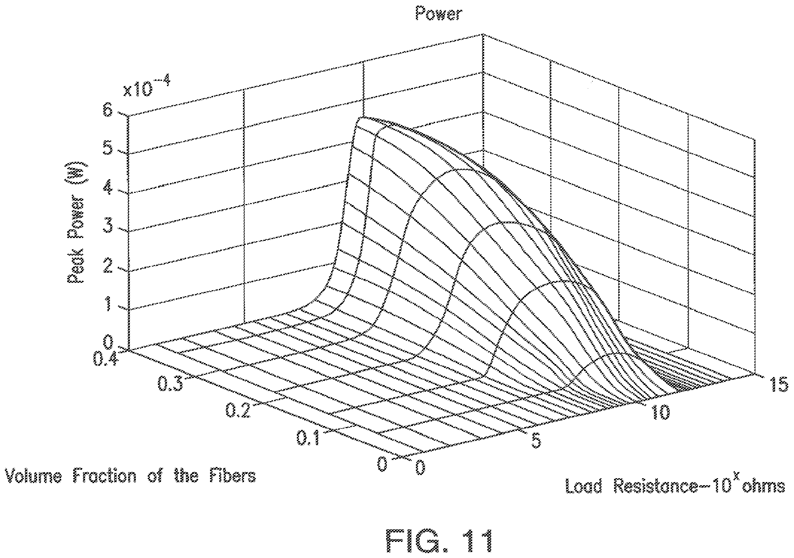

FIG. 11 illustrates the influence of fiber volume fraction and load resistance for an implant with a 120 mm$^2$ cross-sectional area, and thickness of 20 mm with fiber aspect ratio of 100. This implant size and shape can produce a peak power of 0.47 mW at a volume fraction of 31% and load resistance of 8.5 G$\Omega$.

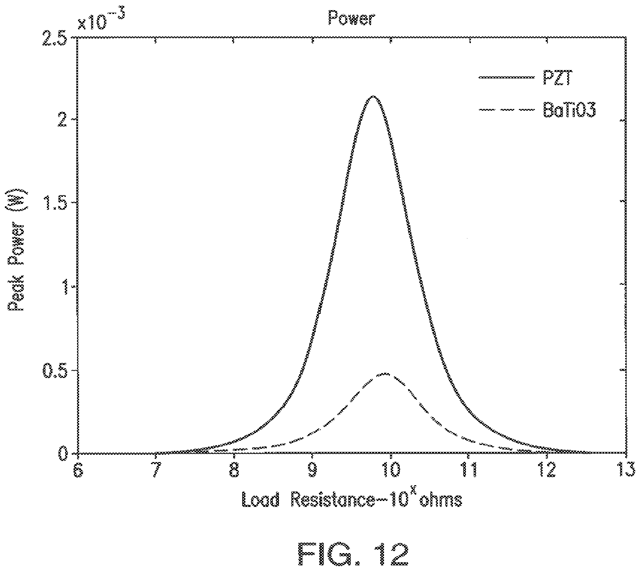

The analysis performed above was conducted for all sets of matrix and fiber materials. It was found that the same trends were present, and that peak performance was generated in each composite for the same specimen area, thickness, fiber aspect ratio, and fiber volume fraction. Therefore, using the implant geometry and fiber variables from the preceding analysis, the effects of using different materials were then compared by using the fiber and matrix variables given in Table 1. The power generated was then plotted versus load resistance as shown in FIG. 12. The PZT - PEEK composite generated a peak power of 2.1 mW, compared to the $BaTiO_3$ - PEEK composite which generated 0.47 mW.

As the $BaTiO_3$ fibers were capable of producing an average rms power approximately 2.4 times the maximum power that is needed for clinical use and as $BaTiO_3$ materials are currently used in FDA approved implants, this fiber type was used to analyze the remaining matrix variables. The results for matrix materials showed no meaningful difference (<11%) in power generation between the highest and lowest output. Similar trends were seen for PZT fibers embedded in the various matrix materials.

Discussion

According to this analysis, an implant comprising a 1-3 structured composite of $BaTiO_3$ fibers and a PEEK matrix is suitably able to generate 2.4 times more power than the maximum currently used to stimulate bone growth. 0-3 composite equations were used to calculate the elastic modulus and electrical resistivity; however, the composites are actually quasi-1-3 composites. The 1-3 composite equations are believed to overestimate the electrical outputs. Therefore, the 0-3 composite equations provide a conservative estimate for these values, providing a minimum baseline for the material's electrical generation.

A 31% fiber volume fraction yielded the largest peak power output for a 20 mm thick implant. Unlike spherical particles, for which theoretical models show a steadily increasing power output with volume fraction (16), the aligned fiber piezoelectric composite output does not exhibit a constantly increasing correlation to volume fraction and instead peaks at an intermediate value. This relationship is due to the variations in piezoelectric and dielectric constant created by the aligned fibers of the 1-3 composite. At low volume fractions, the $d_{33}$ value increases rapidly with increasing volume fractions, but plateaus at a relatively low volume fraction, while the dielectric constant of the composite steadily increases with increasing volume fraction (12). The theoretical model demonstrates that power generation increases with higher $d_{33}$ values, but decreases with increasing fiber dielectric constant. Thus, the peak power output does not have a direct relationship with fiber volume fraction.

Designing an implant that is thick with a small cross-sectional area is one way of increasing overall piezoelectric implant power output. Since the applied force magnitude remains constant, a smaller implant cross-sectional area results in increased implant stress, thus yielding higher power generation. However, the fusion cage must still be able to provide mechanical support for the spine while the vertebrae are fusing and not pose a risk for endplate subsidence. The grade of PEEK that is used in spinal implants has a fatigue strength of 60 MPa, and a compressive strength of 118 MPa, considerably larger than the 4.2 MPa stress level predicted in this theoretical analysis (31). A PEEK composite implant with typical geometry and a cross-sectional area of 120 mm$^2$ is predicted to have a fatigue limit of 7.2 kN and should survive compressive loads up to 14.2 kN, much larger than the anticipated *in vivo* loads.

Peak power for a monolithic piezoelectric composite occurs at a load resistance of 8.5 GΩ, many orders of magnitude higher than the tissue resistance found *in vivo* (0-40 kΩ). A piezoelectric spinal fusion implant may require additional energy harvesting circuitry with a load resistance of 8.5 GΩ and deliver the electricity generated to the desired fusion site. An alternative design utilizes multiple embedded electrodes to reduce the optimal load resistance. This method was proven to be effective for bulk piezoceramics, and is anticipated to work for composite materials as well (18).

The piezoelectric implant parameters found to generate optimal power using a structured 1-3 $BaTiO_3$ fiber and PEEK matrix are summarized in Table 3.

Table 3. Variables that generate maximum power for a BaTiO3 - PEEK spinal fusion cage.

| | **Implant Variables** | **Value for Maximum Power** |
|---|---|---|
| **Fiber Variables** | Volume Fraction | 31% |
| | Aspect Ratio | 100 |
| **Implant Geometry** | Cross-sectional Area | 120 mm$^2$ |
| | Thickness | 20 mm |
| **Circuit Variable** | Load Resistance | 8.5 GΩ |
| **Materials** | Fiber | BaTiO3 |
| | Matrix | PEEK |

A piezoelectric composite spinal implant with these specifications suitably generates an average rms power of 0.33 mW, which is 2.4 times greater than the target power of 0.14 mW. A piezoelectric composite spinal fusion implant suitably delivers a higher current density than existing electrical stimulation devices, thereby speeding bone growth (14, 32). However, if a lower constant dose is required, the excess power generated during patient activity could be stored and distributed as needed when the patient is inactive. In addition, the generation of excess power means the piezoelectric composite implant could still effectively be utilized with cross-sectional areas up to 275 $mm^2$, or thicknesses as small as 9 mm, all while still generating the target constant power of 0.14 mW.

Conclusion

The piezoelectric spinal fusion cage analyzed in this study suitably increases success rates of spinal fusion, particularly in the difficult to fuse patient population. This design fills a large unmet need in the medical community due to the low success rates of current spinal fusion methods in patients with compromised bone fusing ability. Unlike other bone growth stimulants, the piezoelectric spinal implant described herein would not add additional expense, instrumentation, or prolong the implantation procedure greatly, and is predominantly independent of patient compliance. A piezoelectric spinal implant would simply replace the interbody device currently used in the surgery and utilize the patient's own movement to help stimulate bone growth. Based on the model developed for piezoelectric composites, an implant made of $BaTiO_3$ and PEEK suitably generates sufficient power to improve the rate and quantity of bone growth, thereby increasing fusion success rates, thus reducing overall patient care costs.

References

1. NINDS. National Institute of Neurological Disorders and Stroke: Low Back Pain Fact Sheet 2011. Available from: http://www.ninds.nih.gov/disorders/backpain/detail_backpain.htm.

2. Kane WJ. Direct current electrical bone growth stimulation for spinal fusion. Spine. 1988;13(3):363.

3. Kucharzyk DW. A controlled prospective outcome study of implantable electrical stimulation with spinal instrumentation in a high-risk spinal fusion population. Spine. 1999;24(5):465.

4. Meril AJ. Direct current stimulation of allograft in anterior and posterior lumbar interbody fusions. Spine. 1994;19(21):2393.

5. Glazer PA, Glazer LC. Electricity: the history and science of bone growth stimulation for spinal fusion. Orthop J Harvard Med School Online. 2002;4:63-7.

6. An HS, Lynch K, Toth J. Prospective comparison of autograft vs. allograft for adult posterolateral lumbar spine fusion: differences among freeze-dried, frozen, and mixed grafts. Journal of Spinal Disorders. 1995;8(2):131.

7. Mooney V. A randomized double-blind prospective study of the efficacy of pulsed electromagnetic fields for interbody lumbar fusions. Spine. 1990; 15(7):708-12.

8. Carragee EJ, Hurwitz EL, Weiner BK. A critical review of recombinant human bone morphogenetic protein-2 trials in spinal surgery: emerging safety concerns and lessons learned. The Spine Journal. 2011;11(6):471-91.

9. Epstein NE. Pros, cons, and costs of INFUSE in spinal surgery. Surgical neurology international. 2011;2.

10. Epstein NE, Schwall GS. Costs and frequency of "off-label" use of INFUSE for spinal fusions at one institution in 2010. Surgical neurology international. 2011;2.

11. Van den Ende D, Bory B, Groen W, Van der Zwaag S. Improving the d33 and g33 properties of 0-3 piezoelectric composites by dielectrophoresis. Journal of Applied Physics. 2010;107(2):024107--8.

12. Van den Ende D, Van Kempen S, Wu X, Groen W, Randall C, van der Zwaag S. Dielectrophoretically structured piezoelectric composites with high aspect ratio piezoelectric particles inclusions. Journal of Applied Physics. 2012;111:124107.

13. Toth JM, Seim III HB, Schwardt JD, Humphrey WB, Wallskog JA, Turner AS. Direct current electrical stimulation increases the fusion rate of spinal fusion cages. Spine. 2000;25(20):2580.

14. Cook SD, Patron LP, Christakis PM, Bailey KJ, Banta C, Glazer PA. Direct current stimulation of titanium interbody fusion devices in primates. The Spine Journal. 2004;4(3):300-11.

15. Biomet. Implantable Spinal Fusion Stimulators Physician's Manual & Full Prescribing Information SpF PLUS-Mini, SpF - XL IIb 2009. Available from: http://www.biomet.com/spine/getFile.cfm?id=2889&rt=inline.

16. Tobaben N. Development of a Novel Piezoelectric Implant to Improve the Success Rate of Spinal Fusion: University of Kansas; 2012.

17. Platt SR, Farritor S, Garvin K, Haider H. The use of piezoelectric ceramics for electric power generation within orthopedic implants. Mechatronics, IEEE/ASME Transactions on. 2005;10(4):455-61.

18. Platt SR, Farritor S, Haider H. On low-frequency electric power generation with PZT ceramics. Mechatronics, IEEE/ASME Transactions on. 2005;10(2):240-52.

19. Roundy SJ. Energy scavenging for wireless sensor nodes with a focus on vibration to electricity conversion: University of California; 2003.

20. Wang M, Pan N. Predictions of effective physical properties of complex multiphase materials. Materials Science and Engineering: R: Reports. 2008;63(1):1-30.

21. Cappozzo A. Compressive loads in the lumbar vertebral column during normal level walking. Journal of Orthopaedic Research. 1983;1(3):292~ 301.

22. Cheng C, Chen H, Chen C, Lee S. Influences of walking speed change on the lumbosacral joint force distribution. Biomedical Materials and Engineering. 1998;8:155-66.

23. Cromwell R, Schultz AB, Beck R, Warwick D. Loads on the lumbar trunk during level walking. Journal of Orthopaedic Research. 1989;7(3):371-7.

24. Khoo B, Goh J, Bose K. A biomechanical model to determine lumbosacral loads during single stance phase in normal gait. Medical Engineering & Physics. 1995;17(1):27-35.

25. Tsuang YH, Chiang YF, Hung CY, Wei HW, Huang CH, Cheng CK. Comparison of cage application modality in posterior lumbar interbody fusion with posterior instrumentation-A finite element study. Medical engineering & physics. 2009;31(5):565-70.

26. Walpole SC, Prieto-Merino D, Edwards P, Cleland J, Stevens G, Roberts I. The weight of nations: an estimation of adult human biomass. BMC Public Health. 2012;12(1):439.

27. Yoshida T, Kim WC, Kawamoto K, Hiroshima T, Oka Y, Kubo T. Measurement of bone electrical impedance in fracture healing. Journal of Orthopaedic Science. 2009;14(3):320-9.

28. Lei A, Xu R, Thyssen A, Stoot A, Christiansen T, Hansen K, et al., editors. MEMS-based thick film PZT vibrational energy harvester. Micro Electro Mechanical Systems (MEMS), 2011 IEEE 24th International Conference, 2011;125-128,

29. Green S. PEEK-Optima Polymer in the Implantable Medical Device Industry. Invibio Inc, Lancashire, United Kingdom, undated.

30. Wilson SA. 1999. Electric-field structuring of piezoelectric composite materials. PhD Dissertation, Cranfield University, http://dspace.lib.cranfield.ac.uk/handle/1826/3373.

31. Ltd I. Typical Material Properties (Granular) 2004. Available from: http://calvaryspine.com/pdfs/Peek_Optima_Data_Sheet.pdf.

32. Dejardin LM, Kahanovitz N, Amoezky SP, Simon BJ. The effect of varied electrical current densities on lumbar spinal fusions in dogs. The Spine Journal. 2001;1(5):341-7.

It will be readily apparent to one of ordinary skill in the relevant arts that other suitable modifications and adaptations to the methods and applications described hein can be made without departing from the scope of any of the embodiments.

It is to be understood that while certain embodiments have been illustrated and described herein, the claims are not to be limited to the specific forms or arrangement of parts described and shown. In the specification, there have been disclosed illustrative embodiments, and although specific terms are employed, they are used in a generic and descriptive sense only and not for purposes of limitation. Modifications and variations of the embodiments are possible in light of the above teachings. It is therefore to be understood that the embodiments may be practiced otherwise than as specifically described.

U.S. GOVERNMENT RIGHTS

This invention was made with government support under grant no. 1248377 awarded by the National Science Foundation. The government has certain rights in the invention.

## Claims

1. A method of making a piezoelectric composite, the method comprising:

   a) preparing a polymerizable matrix;
   b) dispersing a plurality of piezoelectric particles in the polymerizable matrix to generate a dispersion;
   c) shaping the dispersion;
   d) inducing an electric polarization in the piezoelectric particles in the shaped dispersion by applying a cyclic hydrostatic pressure, wherein at least 40% of the piezoelectric particles form chains as a result of the induction of the electric polarization;

e) applying an electric field in a direction to the shaped dispersion at the same frequency with the cyclic hydrostatic pressure; and

f) curing the dispersion,

wherein the inducing in d) and the applying an electric field in e) occur simultaneously, and wherein at least about 70% of the chains are aligned to within about ±10 degrees of the direction of the applied electric field.

2. The method of claim 1, wherein the polymerizable matrix comprises:

   i. a thermoset polymer, copolymer and/or monomer;
   ii. a thermoplastic polymer, copolymer and/or monomer; or
   iii. a thermoset/thermoplastic polymer or copolymer blend.

3. The method of any of the preceding claims, wherein the piezoelectric particles exhibit a Perovskite crystalline structure.

4. The method of any of the preceding claims, wherein the piezoelectric particles are selected from the group consisting of particles of barium titanate, particles of hydroxyapatite, particles of apatite, particles of lithium sulfate monohydrate, particles of sodium potassium niobate, particles of quartz, particles of lead zirconium titanate (PZT), particles of tartaric acid and poly(vinylidene difluoride) fibers.

5. The method of any of the preceding claims, wherein the shaping comprises injection molding, extrusion, compression molding, blow molding or thermoforming.

6. The method of any of the preceding claims, wherein the inducing an electric polarization comprises applying an electric field in a direction to the shaped dispersion, wherein applying an electric field comprises applying a field with a frequency of about 1 kHz to about 10 kHz and a field strength of about 1 Volt/mm to about 1 kV/mm.

7. The method according to any of the preceding claims, wherein at least about 80%, preferably at least about 90%, of the chains are aligned to within about ±10 degrees of the direction of the applied electric field.


**Patentansprüche**

1. Verfahren zur Herstellung eines piezoelektrischen Verbundwerkstoffs, wobei das Verfahren umfasst:

   a) Vorbereiten einer polymerisierbaren Matrix;
   b) Dispergieren einer Vielzahl von piezoelektrischen Partikeln in der polymerisierbaren Matrix, um eine Dispersion zu erzeugen;
   c) Formen der Dispersion;
   d) Induzieren einer elektrischen Polarisation in den piezoelektrischen Partikeln in der geformten Dispersion durch Anlegen eines zyklischen hydrostatischen Drucks, wobei mindestens 40% der piezoelektrischen Partikel Ketten bilden, als Ergebnis der Induktion der elektrischen Polarisation;
   e) Anlegen eines elektrischen Feldes in einer Richtung an die geformte Dispersion mit der gleichen Frequenz wie der zyklische hydrostatische Druck; und
   f) Aushärten der Dispersion,
   wobei das Induzieren in d) und das Anlegen eines elektrischen Feldes in e) gleichzeitig stattfinden, und wobei mindestens etwa 70% der Ketten innerhalb von etwa ±10 Grad zur Richtung des angelegten elektrischen Feldes ausgerichtet sind.

2. Verfahren nach Anspruch 1, wobei die polymerisierbare Matrix umfasst:

   i. ein duroplastisches Polymer, Copolymer und/oder Monomer;
   ii. ein thermoplastisches Polymer, Copolymer und/oder Monomer; oder
   iii. eine duroplastische/thermoplastische Polymer- oder Copolymermischung.

3. Verfahren nach einem der vorangegangenen Ansprüche, wobei die piezoelektrischen Partikel eine Perowskit-Kristallstruktur aufweisen.

**4.** Verfahren nach einem der vorangegangenen Ansprüche, wobei die piezoelektrischen Partikel ausgewählt sind aus der Gruppe bestehend aus Partikel aus Bariumtitanat, Partikel aus Hydroxylapatit, Partikel aus Apatit, Partikel aus Lithiumsulfat-Monohydrat, Partikel aus Natriumkaliumniobat, Partikel aus Quarz, Partikel aus Bleizirkoniumtitanat (PZT), Partikel aus Weinsäure und Poly(vinylidendifluorid)-Fasern.

**5.** Verfahren nach einem der vorangegangenen Ansprüche, wobei das Formen Spritzgießen, Fließpressen, Formpressen, Blasformen oder Thermoformen umfasst.

**6.** Verfahren nach einem der vorangegangenen Ansprüche, wobei das Induzieren einer elektrischen Polarisation das Anlegen eines elektrischen Feldes in einer Richtung auf die geformte Dispersion umfasst, wobei das Anlegen eines elektrischen Feldes das Anlegen eines Feldes mit einer Frequenz von etwa 1 kHz bis etwa 10 kHz und einer Feldstärke von etwa 1 Volt/mm bis etwa 1 kV/mm umfasst.

**7.** Verfahren nach einem der vorangegangenen Ansprüche, wobei mindestens etwa 80%, vorzugsweise mindestens etwa 90% der Ketten innerhalb von etwa ±10 Grad zur Richtung des angelegten elektrischen Feldes ausgerichtet sind.

**Revendications**

**1.** Procédé de fabrication d'un composite piézoélectrique, le procédé comprenant :

a) la préparation d'une matrice polymérisable ;
b) la dispersion d'une pluralité de particules piézoélectriques dans la matrice polymérisable pour générer une dispersion ;
c) le façonnage de la dispersion ;
d) l'induction d'une polarisation électrique dans les particules piézoélectriques dans la dispersion façonnée par application d'une pression hydrostatique cyclique, dans lequel au moins 40 % des particules piézoélectriques forment des chaînes en conséquence de l'induction de la polarisation électrique ;
e) l'application d'un champ électrique dans une direction vers la dispersion façonnée à la même fréquence que la pression hydrostatique cyclique ; et
f) le durcissement de la dispersion,
dans lequel l'induction dans d) et l'application d'un champ électrique dans e) se produisent simultanément, et dans lequel au moins environ 70 % des chaînes sont alignées au plus à environ ±10 degrés par rapport à la direction du champ électrique appliqué.

**2.** Procédé selon la revendication 1, dans lequel la matrice polymérisable comprend :

i. un polymère, copolymère et/ou monomère thermodurcissable ;
ii. un polymère, copolymère et/ou monomère thermoplastique ; ou
iii. un mélange de polymères ou copolymères thermodurcissables/ thermoplastiques.

**3.** Procédé selon l'une quelconque des revendications précédentes, dans lequel les particules piézoélectriques présentent une structure cristalline de pérovskite.

**4.** Procédé selon l'une quelconque des revendications précédentes, dans lequel les particules piézoélectriques sont choisies dans le groupe constitué de particules de titanate de baryum, particules d'hydroxyapatite, particules d'apatite, particules de sulfate de lithium monohydraté, particules de niobate de sodium-potassium, particules de quartz, particules de titano-zirconates de plomb(PZT), particules d'acide tartrique et fibres de poly(difluorure de vinylidène).

**5.** Procédé selon l'une quelconque des revendications précédentes, dans lequel le façonnage comprend un moulage par injection, une extrusion, un moulage par compression, un moulage par soufflage ou un thermoformage.

**6.** Procédé selon l'une quelconque des revendications précédentes, dans lequel l'induction d'une polarisation électrique comprend l'application d'un champ électrique dans une direction vers la dispersion façonnée, dans lequel l'application d'un champ électrique comprend l'application d'un champ ayant une fréquence d'environ 1 kHz à environ 10 kHz et une intensité de champ d'environ 1 volt/mm à environ 1 kV/mm.

**7.** Procédé selon l'une quelconque des revendications précédentes, dans lequel au moins environ 80 %, de préférence au moins environ 90 %, des chaînes sont alignées au plus à environ $\pm 10$ degrés par rapport à la direction du champ électrique appliqué.

FIG. 1B

FIG. 1A

EP 2 859 599 B1

```
┌─────────────────────────────────┐
│  Prepare  Polymerizable  Matrix │
└─────────────────────────────────┘
                 │
                 ▼
┌─────────────────────────────────┐
│ Disperse  Piezoelectric  Particles │
└─────────────────────────────────┘
                 │
                 ▼
┌─────────────────────────────────┐
│       Shape  Dispersion         │
└─────────────────────────────────┘
                 │
                 ▼
┌─────────────────────────────────┐
│            Induce               │
│           Electric              │
│          Polarization           │
└─────────────────────────────────┘
                 │
                 ▼
┌─────────────────────────────────┐
│        Cure  Dispersion         │
└─────────────────────────────────┘
```

FIG. 2A

FIG. 2B

FIG. 3B

302

$F_{DEP}$

204

FIG. 3A

302

$F_{DEP}$

204

FIG. 4A

EP 2 859 599 B1

FIG. 4B

FIG. 5

EP 2 859 599 B1

FIG. 6

FIG. 7

FIG. 8

FIG. 9

EP 2 859 599 B1

FIG. 10

EP 2 859 599 B1

FIG. 11

FIG. 12

EP 2 859 599 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4407054 A

- US 6143035 A

### Non-patent literature cited in the description

- Improving the d33 and g33 properties of 0-3 piezoelectric composites by dielectrophoresis. *Journal of Applied Physics,* vol. 107 (2), 24107-1, 24107-8
- Low Back Pain Fact Sheet. National Institute of Neurological Disorders and Stroke, 2011
- KANE WJ. Direct current electrical bone growth stimulation for spinal fusion. *Spine,* 1988, vol. 13 (3), 363
- KUCHARZYK DW. A controlled prospective outcome study of implantable electrical stimulation with spinal instrumentation in a high-risk spinal fusion population. *Spine,* 1999, vol. 24 (5), 465
- MERIL AJ. Direct current stimulation of allograft in anterior and posterior lumbar interbody fusions. *Spine,* 1994, vol. 19 (21), 2393
- GLAZER PA ; GLAZER LC. Electricity: the history and science of bone growth stimulation for spinal fusion. *Orthop J Harvard Med School Online,* 2002, vol. 4, 63-7
- AN HS ; LYNCH K ; TOTH J. Prospective comparison of autograft vs. allograft for adult posterolateral lumbar spine fusion: differences among freeze-dried, frozen, and mixed grafts. *Journal of Spinal Disorders,* 1995, vol. 8 (2), 131
- MOONEY V. A randomized double-blind prospective study of the efficacy of pulsed electromagnetic fields for interbody lumbar fusions. *Spine,* 1990, vol. 15 (7), 708-12
- CARRAGEE EJ ; HURWITZ EL ; WEINER BK. A critical review of recombinant human bone morphogenetic protein-2 trials in spinal surgery: emerging safety concerns and lessons learned. *The Spine Journal.,* 2011, vol. 11 (6), 471-91
- EPSTEIN NE. Pros, cons, and costs of INFUSE in spinal surgery. *Surgical neurology international,* 2011, vol. 2
- EPSTEIN NE ; SCHWALL GS. Costs and frequency of "off-label" use of INFUSE for spinal fusions at one institution. *Surgical neurology international. 2011,* 2010, 2

- VAN DEN ENDE D ; BORY B ; GROEN W ; VAN DER ZWAAG S. Improving the d33 and g33 properties of 0-3 piezoelectric composites by dielectrophoresis. *Journal of Applied Physics,* 2010, vol. 107 (2), 024107-8
- VAN DEN ENDE D ; VAN KEMPEN S ; WU X ; GROEN W ; RANDALL C ; VAN DER ZWAAG S. Dielectrophoretically structured piezoelectric composites with high aspect ratio piezoelectric particles inclusions. *Journal of Applied Physics,* 2012, vol. 111, 124107
- TOTH JM ; SEIM III HB ; SCHWARDT JD ; HUMPHREY WB ; WALLSKOG JA ; TURNER AS. Direct current electrical stimulation increases the fusion rate of spinal fusion cages. *Spine,* 2000, vol. 25 (20), 2580
- COOK SD ; PATRON LP ; CHRISTAKIS PM ; BAILEY KJ ; BANTA C ; GLAZER PA. Direct current stimulation of titanium interbody fusion devices in primates. *The Spine Journal,* 2004, vol. 4 (3), 300-11
- Biomet. Implantable Spinal Fusion Stimulators Physician's Manual & Full Prescribing Information SpF PLUS-Mini. 2009
- TOBABEN N. Development of a Novel Piezoelectric Implant to Improve the Success Rate of Spinal Fusion. University of Kansas, 2012
- PLATT SR ; FARRITOR S ; GARVIN K ; HAIDER H. The use of piezoelectric ceramics for electric power generation within orthopedic implants. *Mechatronics, IEEE/ASME Transactions on,* 2005, vol. 10 (4), 455-61
- PLATT SR ; FARRITOR S ; HAIDER H. On low-frequency electric power generation with PZT ceramics. *Mechatronics, IEEE/ASME Transactions on,* 2005, vol. 10 (2), 240-52
- ROUNDY SJ. Energy scavenging for wireless sensor nodes with a focus on vibration to electricity conversion. University of California, 2003
- WANG M ; PAN N. Predictions of effective physical properties of complex multiphase materials. *Materials Science and Engineering: R: Reports.,* 2008, vol. 63 (1), 1-30

- **CAPPOZZO A.** Compressive loads in the lumbar vertebral column during normal level walking. *Journal of Orthopaedic Research,* 1983, vol. 1 (3), 292-301
- **CHENG C ; CHEN H ; CHEN C ; LEE S.** Influences of walking speed change on the lumbosacral joint force distribution. *Biomedical Materials and Engineering,* 1998, vol. 8, 155-66
- **CROMWELL R ; SCHULTZ AB ; BECK R ; WARWICK D.** Loads on the lumbar trunk during level walking. *Journal of Orthopaedic Research.,* 1989, vol. 7 (3), 371-7
- **KHOO B ; GOH J ; BOSE K.** A biomechanical model to determine lumbosacral loads during single stance phase in normal gait. *Medical Engineering & Physics,* 1995, vol. 17 (1), 27-35
- **TSUANG YH ; CHIANG YF ; HUNG CY ; WEI HW ; HUANG CH ; CHENG CK.** Comparison of cage application modality in posterior lumbar interbody fusion with posterior instrumentation-A finite element study. *Medical engineering & physics,* 2009, vol. 31 (5), 565-70
- **WALPOLE SC ; PRIETO-MERINO D ; EDWARDS P ; CLELAND J ; STEVENS G ; ROBERTS I.** The weight of nations: an estimation of adult human biomass. *BMC Public Health,* 2012, vol. 12 (1), 439
- **YOSHIDA T ; KIM WC ; KAWAMOTO K ; HIROSHIMA T ; OKA Y ; KUBO T.** Measurement of bone electrical impedance in fracture healing. *Journal of Orthopaedic Science,* 2009, vol. 14 (3), 320-9
- MEMS-based thick film PZT vibrational energy harvester. Micro Electro Mechanical Systems (MEMS). IEEE 24th International Conference, 2011. 2011, 125-128
- **GREEN S.** PEEK-Optima Polymer in the Implantable Medical Device Industry. Invibio Inc
- Electric-field structuring of piezoelectric composite materials. **WILSON SA.** PhD Dissertation. Cranfield University, 1999
- *Ltd I. Typical Material Properties (Granular),* 2004, http://calvaryspine.com/pdfs/Peek_Optima_Data_Sheet.pdf
- **DEJARDIN LM ; KAHANOVITZ N ; AMOEZKY SP ; SIMON BJ.** The effect of varied electrical current densities on lumbar spinal fusions in dogs. *The Spine Journal,* 2001, vol. 1 (5), 341-7